# EUROPEAN PATENT APPLICATION

(11) **EP 1 754 739 A1**
(43) Date of publication of application: **21.02.2007**
(21) Application number: 05741155.5
(22) Date of filing: 17.05.2005
(51) Int. Cl.: C08G 73/10, G02B 6/12

(54) **DEUTERATED POLYIMIDES AND DERIVATIVES THEREOF**

(30) Priority: 21.05.2004 JP 2004151209
(71) Applicant: Wako Pure Chemical Industries, Ltd., Osaka-shi, Osaka 540-8605 (JP)
(72) Inventor: MUTO, Kazushige, Saitama 350-1101 (JP); MAESAWA, Tsuneaki, Saitama 350-1101 (JP); ITO, Nobuhiro, Saitama 350-1101 (JP); WATAHIKI, Tsutomu, Saitama 350-1101 (JP); HIROTA, Kosaku, Gifu 502-0003 (JP); SAJIKI, Hironao, Gifu 502-0823 (JP)
(74) Representative: Kaiser, Jürgen
(86) International application number: PCT/JP2005/008984
(87) International publication number: WO 2005/113646

(57) **Abstract**

The object of the present invention is to provide a polyimide compound useful as a raw material of a polymer for an optical waveguide that has excellent transparency and heat resistance, low moisture absorption, a small optical transmission loss, a high refractive index and good adhesion to a base material or a substrate. The present invention is inventions of a method for producing a deuterated polyamic acid compound represented by the general formula [2]: 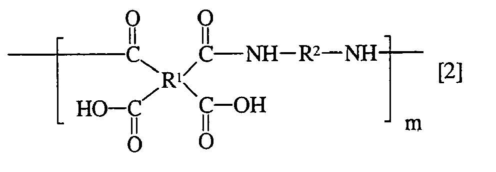 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and m indicates an integer not less than 1,
which comprises reacting an acid anhydride represented by the general formula [3]: 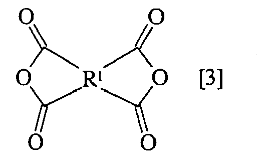 wherein R¹ has the same meaning as above, with a deuterated diamine compound represented by the general formula [4]:

H₂N-R²-NH₂ [4]

wherein R² has the same meaning as above; a method for producing a deuterated polyimide compound represented by the general formula [1]: 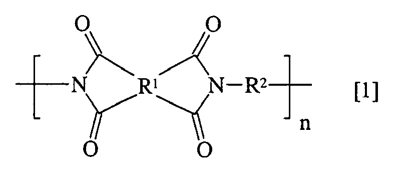 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1, which comprises subjecting thus obtained deuterated polyamic acid compound to a ring closure reaction; a method for producing a deuterated polyimide compound represented by the general formula [1] which comprises reacting an acid anhydride represented by the above general formula [3] with a deuterated diamine compound represented by the above general formula [4] and then subjecting the reaction product to a ring closure reaction; use of a deuterated polyimide compound obtained by the above production methods as a raw material polymer for an optical waveguide; a film for an optical waveguide containing said deuterated polyimide compound; a deuterated polyimide compound; and a deuterated polyamic acid compound.

## Description

### Technical Field

The present invention relates to a new deuterated polyimide compound and its derivative useful as a raw material of an optical waveguide that is excellent in heat resistance, transparency, adhesion to a substrate and processability.

### Background Art

An optical waveguide made of glass has been conventionally used as a material for an optical waveguide. However, temperature processing over 1000°C is required in its production. Therefore, a polymer material has been searched for as a material for an optical waveguide instead. Recently, a polyimide compound has been attracting attention as one of polymers for an optical waveguide. However, a light hydrogen polyimide compound has many C-H bonds, and thus a polymer containing said compound is poor in transparency and causes a large optical transmission loss in a near-infrared region. Therefore, such a polyimide compound is not suitable for a raw material of an optical waveguide to be used for a high-capacity and high-speed transmission system. In these situations, a fluorinated polyimide compound formed by partially substituting light hydrogen atoms of such a light hydrogen polyimide compound with fluorine atoms has been studied as a polymer for an optical waveguide (Patent Literatures 1 to 8). A fluorinated polyimide compound has been drawing attention as a raw material polymer of an optical waveguide because of its small optical transmission loss in a near-infrared region and its excellent heat resistance and low moisture absorption. However it has a problem that when used as a core material of an optical waveguide, its refractive index is so low that clad materials having refractive index matching the core material are required. Therefore, subsequently clad materials are limited. The fluorinated polyimide compound also has problems in that it causes poor transparency and a large optical transmission loss due to C-H bonds at a low fluorination rate as mentioned above, whereas at a high fluorination rate, it suffers poor adhesion to a base material or a substrate due to low surface tension of said compound resulting in difficulty of processing such as coating, and also poor adhesion of a film made of said compound resulting in poor film characteristics and a fragile film.

In these situations, a polyimide compound useful as a raw material of a polymer for an optical waveguide that has excellent transparency and heat resistance, low moisture absorption, a small optical transmission loss, a high refractive index and good adhesion to a base material or a substrate has been desired to come into being.

On the other hand, there are various literatures such as a literature (Non Patent Literature 1) investigating the relation between imidation temperature, imidation rate and interface diffusion distance using a deuterated polyamic ester, a literature (Non Patent Literature 2) investigating interface diffusion on the surface of a deuterated polyamic acid and a polyimide film by an ion beam method (note: it does not specify what part is deuterated) and a literature (Non Patent Literature 3) investigating by ion beam analysis the dynamics of a deuterated polyamic ester of which the ester portion only is deuterated.

Patent Literature 1: JP-A-2-281037
Patent Literature 2: JP-A-4-8734
Patent Literature 3: JP-A-4-9807
Patent Literature 4: JP-A-5-164929
Patent Literature 5: JP-A-6-51146
Patent Literature 6: JP-A-2001-342203
Patent Literature 7: JP-A-2002-37885
Patent Literature 8: JP-A-2003-160664
Non Patent Literature 1: POLYMER (1997), 38 (20), 5073-5078
Non Patent Literature 2: POLYMER (1992), 33 (16), 3382-3387
Non Patent Literature 3: POLYMER (1990), 31 (3), 520-523

### Disclosure of the Invention

### Problem to be Solved by the Invention

The subject of the present invention is to provide a polyimide compound useful as a raw material of a polymer for an optical waveguide that has excellent transparency and heat resistance, low moisture absorption, a small optical transmission loss, a high refractive index and good adhesion to a base material or a substrate.

### Means to Solve the Subject

In one aspect, the present invention is an invention of a method for producing a deuterated polyamic acid compound represented by the general formula [2]: 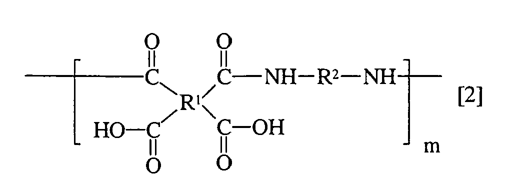 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and m indicates an integer not less than 1,
which comprises reacting an acid anhydride represented by the general formula [3]:

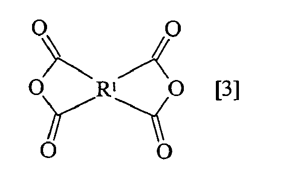

wherein R¹ has the same meaning as above, with a deuterated diamine compound represented by the general formula [4]:

H₂N-R²-NH₂ [4]

wherein R² has the same meaning as above;
A method for producing a deuterated polyimide compound represented by the general formula [1]:

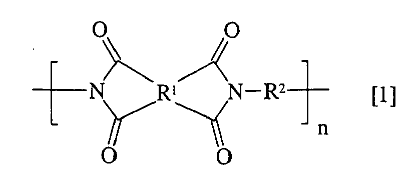

wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
which comprises subjecting thus obtained deuterated polyamic acid compound to a ring closure reaction;
and a method for producing a deuterated polyimide compound represented by the general formula [1] which comprises reacting an acid anhydride represented by the above general formula [3] with a deuterated diamine compound represented by the above general formula [4] and then subjecting the reaction product to a ring closure reaction.

In a further aspect, the present invention is use of a deuterated polyimide compound obtained by the above production methods as a raw material polymer for an optical waveguide, and a film for an optical waveguide containing said deuterated polyimide compound.

In a still further aspect, the present invention is a deuterated polyimide compound represented by the general formula [1]:

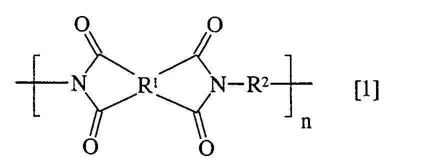

wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
and a deuterated polyamic acid compound represented by the general formula [2]:

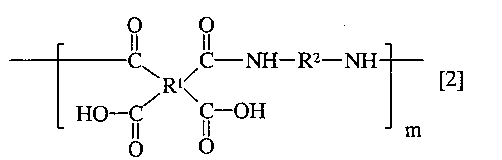

wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and m indicates an integer not less than 1.

### Effect of the Invention

A deuterated polyimide compound of the present invention is a polymer of a high deuteration ratio and therefore useful as a raw material of a polymer for an optical waveguide that has excellent transparency and heat resistance, low moisture absorption, a small optical transmission loss, a high refractive index and good adhesion to a base material or a substrate. A deuterated polyamic acid compound of the present invention is a very useful compound because it enables the above deuterated polyimide compound of a high deuteration ratio to be obtained by easy operations.

### Best Mode for Carrying-out of the Invention

In the present specification, a hydrogen atom generically means a light hydrogen atom and a heavy hydrogen atom. The heavy hydrogen atom means a deuterium (D) or a tritium (T). In the present invention, a ratio of hydrogen atoms substituted by heavy hydrogen atoms to the total hydrogen atoms contained in a compound is referred to as a deuteration ratio.

In the production method of the present invention, the tetravalent alicyclic hydrocarbon group that may have a heavy hydrogen atom indicated by R¹ in an acid anhydride represented by the general formula [3] to be used, a deuterated polyimide compound represented by the general formula [1] to be obtained and a deuterated polyamic acid compound represented by the general formula [2] includes a group that has 4 direct-linkages at optional positions of an alicyclic hydrocarbon ring that may have a heavy hydrogen atom. Specific examples of the alicyclic hydrocarbon ring includes a ring of having 4 to 12 carbon atoms, preferably 4 to 8 carbon atoms, more preferably 4 to 6 carbon atoms and a monocyclic, polycyclic and spiro ring as well as the above rings in which 2 separate carbon atoms are cross-linked by an alkenylene group having 2 to 4 carbon atoms (e.g., a vinylene group, a propenylene group and a butenylene group) or an alkylene group having 1 to 4 carbon atoms (e.g., a methylene group, an ethylene group, a trimethylene group, a propylene group and a tetramethylene group). Specific examples of the alicyclic hydrocarbon ring includes, for example, a monocyclic ring such as cyclobutane, cyclopentane, cyclohexane, cycloheptane, cuclooctane, cyclononane, cyclodecane, cycloundecane and cyclododecane; a crosslinked ring such as norbornane and bicycle[2,2,2]oct-2-ene; or a polycyclic and spiro ring, formed by bonding an optional number of the above rings at optional positions. These alicyclic hydrocarbon rings may have further 1 to 10, preferably 1 to 5, more preferably 1 to 3 alkyl substituents. The above alkyl substituent may be straight chained or branched and includes one having generally 1 to 6, preferably 1 to 3, more preferably 1 or 2, and still more preferably 1 carbon atom, which is specifically exemplified by, a methyl group, an ethyl group, a n-propyl group, an isopropyl group, a n-butyl group, an isobutyl group, a sec-butyl group, a tert-butyl group, a n-pentyl group, an isopentyl group, a sec-pentyl group, a tert-pentyl group, a neopentyl group, a n-hexyl group, an isohexyl group, a sec-hexyl group and a tert-hexyl group.

The above alicyclic hydrocarbon ring as well as the above alkyl substituent may not have a heavy hydrogen atom, but those having a heavy hydrogen atom are preferable. The more heavy hydrogen atoms they have, the more preferable they are.

4 direct-linkages in the tetravalent alicyclic hydrocarbon group preferably has a pair of 2 direct-linkages at 2 adjacent carbon atoms in the alicyclic hydrocarbon ring, more preferably has 2 pairs of the direct-linkages at 2 pairs of adjacent carbon atoms positioned most distantly from one another in the alicyclic hydrocarbon ring, and still more preferably has 2 pairs of the direct-linkages at 2 pairs of adjacent carbon atoms positioned symmetrically in the alicyclic hydrocarbon ring.

Specific examples of the above tetravalent alicyclic hydrocarbon group preferably includes, for example, one shown below,

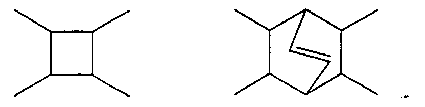

The tetravalent aromatic hydrocarbon group that may have a heavy hydrogen atom indicated by R¹ may be monocyclic or polycyclic and includes a group having 4 direct-linkages at optional positions in the aromatic hydrocarbon ring that may have a heavy hydrogen atom, and a group formed by linking 2 to 6 aromatic hydrocarbon rings through a direct-linkage, an alkylene group, an oxygen atom, a sulfur atom, a sulfonyl group, a carbonyl group, a group obtained by combining the above groups and the like. The tetravalent aromatic hydrocarbon groups include specifically, for example, tetravalent benzene, tetravalent naphthalene, tetravalent anthracene, tetravalent chrysene and a group represented the following general formula [5]:

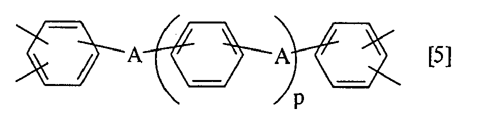

(wherein A indicates a direct-linkage, an alkylene group, an oxygen atom, a sulfur atom, a sulfonyl group, a carbonyl group or a group obtained by combining the above groups; p indicates an integer of 0 to 5).

The above tetravalent aromatic hydrocarbon group having 4 direct-linkages preferably has a pair of 2 direct-linkages at 2 adjacent carbon atoms in any aromatic ring contained in the aromatic hydrocarbon group, more preferably has 2 pairs of the direct-linkages at 2 pairs of adjacent carbon atoms positioned most distantly from one another in all aromatic rings contained in the aromatic hydrocarbon group, and still more preferably has 2 pairs of the direct-linkages at 2 pairs of adjacent carbon atoms positioned symmetrically in the aromatic hydrocarbon ring.

The tetravalent aromatic hydrocarbon group may have further 1 to 10, preferably 1 to 5, more preferably 1 to 3 alkyl substituents in an aromatic ring portion thereof. Specific examples of such a group include the same as the alkyl substituents that the above alicyclic hydrocarbon ring may have.

The above tetravalent aromatic hydrocarbon group as well as the above alkyl substituent may not have a heavy hydrogen atom, but those having a heavy hydrogen atom are preferable. The more heavy hydrogen atoms they have, the more preferable they are. The tetravalent aromatic hydrocarbon group, wherein all hydrogen atoms of the aromatic rings thereof are substituted with heavy hydrogen atoms, is preferable, and the tetravalent aromatic hydrocarbon group, wherein all hydrogen atoms thereof are substituted with heavy hydrogen atoms, is particularly preferable.

The alkylene group indicated by A in the general formula [5] includes a straight chained or branched group having 1 to 6 carbon atoms, which are specifically exemplified by, for example, a methylene group, an ethylene group, a n-propylene group, an isopropylene group, a n-butylene group, an isobutylene group, a sec-butylene group, a tert-butylene group, a n-pentylene group, an isopentylene group, a sec-pentylene group, a tert-pentylene group, a neopentylene group, a n-hexylene group, an isohexylene group, a sec-hexylene group and a tert-hexylene group.

The group obtained by combining the above groups, indicated by A includes, for example, a group formed by combining usually 2 to 15, preferably 2 to 10, more preferably 2 to 5 groups selected from the group consisting of the above direct-linkage; alkylene group, oxygen atom, sulfur atom, sulfonyl group and carbonyl group, which is specifically exemplified by, for example, an oxyalkylene group composed of an alkylene group and an oxygen atom that may have the oxygen atom or the alkylene group at the both ends thereof, and a group formed by combining an alkylene group, an oxygen atom and a carbonyl group that has the carbonyloxy groups at the both ends of the alkylene group.

The symbol p indicates an integer of usually 0 to 5, preferably 0 to 2, more preferably 0 or 1, and still more preferably 0.

Preferable specific examples of the above tetravalent aromatic hydrocarbon group include, for example, the following groups.

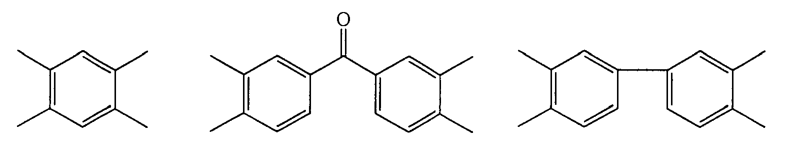

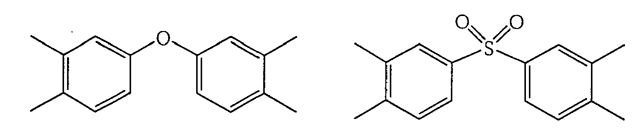

The above tetravalent alicyclic hydrocarbon group and tetravalent aromatic hydrocarbon group, which may have a heavy hydrogen atom indicated by above R¹ may have a fluorine atom. However, the less the number of the fluorine atom contained, the more preferable it is, and the group having no fluorine atom is still more preferable. When the fluorine atom is contained, the number of the fluorine atom is usually 1 to 6, preferably 1 to 3, and more preferably 1.

R¹ in the above compounds represented by the general formulae [1], [2] and [3] is preferably tetravalent aromatic hydrocarbon groups and among them more preferably a group having a monocyclic aromatic ring, especially such a group having a chain of the aromatic rings as represented by the general formula [5]. The aromatic ring has preferably no alkyl group as a substituent. When the aromatic ring has an alkyl group as a substituent, the alkyl group preferably has less carbon atoms.

In the production method of the present invention, the divalent aromatic hydrocarbon group having a heavy hydrogen atom indicated by R² in a deuterated diamine compound represented by the general formula [4] to be used, a deuterated polyimide compound represented by the general formula [1] to be obtained and a deuterated polyamic acid compound represented by the general formula [2] may be monocyclic or polycyclic and includes a group having 2 direct-linkages at optional positions of the aromatic hydrocarbon ring having a heavy hydrogen atom and a group formed by combining 2 to 6 aromatic hydrocarbon rings through, for example, a direct-linkage, an alkylene group that may have an oxygen atom, an oxygen atom, a sulfur atom, a sulfonyl group, a carbonyl group and an alkylene group having carbonyloxy groups at the both ends thereof.

Such divalent aromatic hydrocarbon groups include specifically, for example, divalent benzene, divalent naphthalene, divalent anthracene, divalent chrysene and a group represented by, for example, the following general formula [6]:

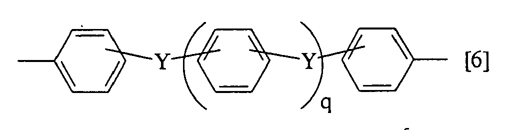

(wherein Y indicates a direct-linkage, an alkylene group, an oxygen atom, a sulfur atom, a sulfonyl group, a carbonyl group or a group obtained by combining the above groups; q indicates an integer of 0 to 5). Said divalent aromatic hydrocarbon group may have further 1 to 10, preferably 1 to 5, more preferably 1 to 3 alkyl substituents in an aromatic ring portion thereof. Specific examples of such a group include the same as the alkyl substituents that the above tetravalent alicyclic hydrocarbon ring and aromatic hydrocarbon ring may have.

The above divalent aromatic hydrocarbon group has at least 1 heavy hydrogen atom on the aromatic ring. Preferable are those having more hydrogen atoms of the aromatic ring thereof substituted with heavy hydrogen atoms, and those having more hydrogen atoms of the groups other than the aromatic ring substituted with heavy hydrogen atoms. More preferable are those having all hydrogen atoms on the aromatic ring thereof substituted with heavy hydrogen atoms. Particularly preferable are the divalent aromatic hydrocarbon groups having all hydrogen atoms therein substituted with heavy hydrogen atoms.

The alkylene group and the group obtained by combining the above groups, indicated by Y in the general formula [6], include the same as the alkylene group indicated by A in the above general formula [5] and the group obtained by combining the above groups.

The symbol q indicates an integer of usually 0 to 5, preferably 0 to 2, more preferably 0 or 1, and still more preferably 0.

The preferable divalent aromatic hydrocarbon group includes specifically, for example, the following groups.

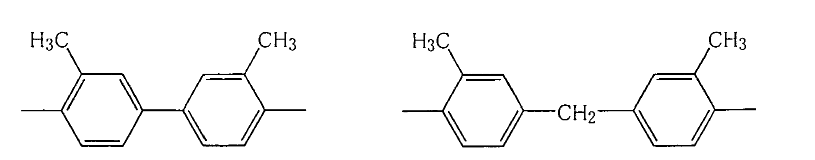

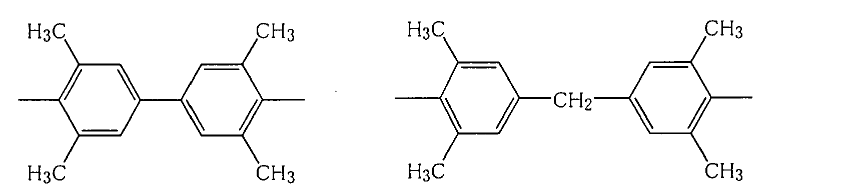

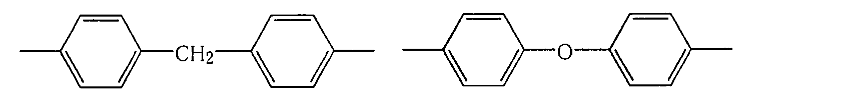

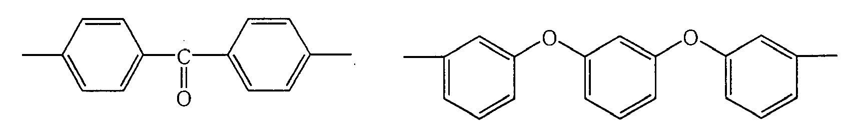

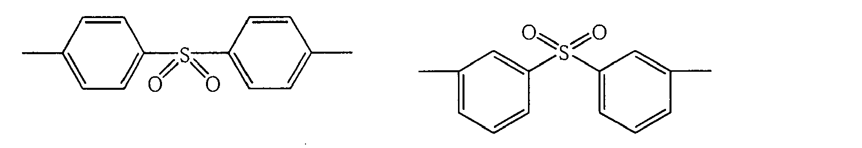

The above divalent aromatic hydrocarbon group having a heavy hydrogen atom, indicated by R² may have a fluorine atom. However, the less the number of the fluorine atom contained, the more preferable it is, and the group having no fluorine atom is still more preferable. When the fluorine atom is contained, the number of the fluorine atom is usually 1 to 6, preferably 1 to 3, and more preferably 1.

Among the divalent aromatic hydrocarbon groups having a heavy hydrogen atom, indicated by R² in the above compounds represented by the general formulae [1], [2] and [4], those having a monocyclic aromatic ring are preferable, and among which those represented by the general formula [6] are more preferable. The aromatic ring has preferably no alkyl group as a substituent. When the aromatic ring has an alkyl group as a substituent, the fewer carbon atoms the alkyl group has, the more preferable it is.

The amount of heavy hydrogen atoms that a divalent aromatic hydrocarbon group indicated by R² in the general formula [4] has is usually not less than 20%, preferably 20 to 100%, more preferably 60 to 100% and still more preferably 80 to 100%, of the amount of hydrogen atoms that the divalent aromatic hydrocarbon group has.

The symbols n and m in the above general formulae [1] and [2] indicate an integer of usually 1 to 10,000, preferably 10 to 3,000, and more preferably 100 to 1,000. The compound represented by the general formula [1] includes a compound having the following structures [8]: 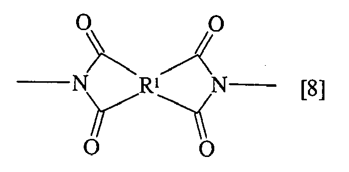 and [9]:

-R²- [9]

(wherein R¹ and R² are the same as the above) at both ends thereof, and the compound represented by the general formula [2] includes a compound having the following structures [10]: 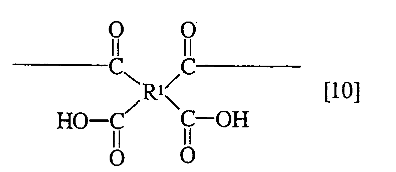 and [11]:

-NH-R²-NH- [11]

(wherein R¹ and R² are the same as the above) at both ends thereof.

Among the above deuterated polyimide compounds represented by the general formula [1], obtained by the method of the present invention, the preferable compound includes a compound represented by the general formula [1']:

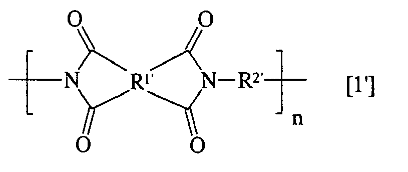

(wherein R¹' indicates a tetravalent alicyclic hydrocarbon group or a tetravalent aromatic hydrocarbon group, which may have a heavy hydrogen atom; R²' indicates a divalent aromatic hydrocarbon group having a heavy hydrogen atom; and n indicates an integer not less than 1, and provided that R¹' and R²' have no fluorine atom).

Among the above deuterated polyamic acid compounds represented by the general formula [2], obtained by the method of the present invention, the preferable compound includes a compound represented by the general formula [2']:

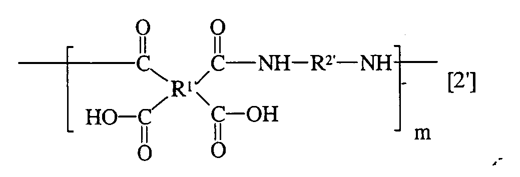

(wherein R¹' indicates a tetravalent alicyclic hydrocarbon group or a tetravalent aromatic hydrocarbon group, which may have a heavy hydrogen atom; R²' indicates a divalent aromatic hydrocarbon group having a heavy hydrogen atom; and m indicates an integer not less than 1, and provided that R¹' and R²' have no fluorine atom).

In the above general formulae [1'] and [2'], the tetravalent alicyclic hydrocarbon group or tetravalent aromatic hydrocarbon group, which may have a heavy hydrogen atom, indicated by R¹' includes the same as tetravalent alicyclic hydrocarbon group or tetravalent aromatic hydrocarbon group, which may have a heavy hydrogen atom, indicated by R¹ in the deuterated polyimide compound represented by the general formulae [1] and [2] excluding the group having a fluorine atom. The divalent aromatic hydrocarbon group having a heavy hydrogen atom indicated by R²' may be monocyclic or polycyclic and includes a group that has 2 direct-linkages at optional positions of an aromatic hydrocarbon ring having a heavy hydrogen atom and does not contain a fluorine atom, and also includes a group formed by combining 2 to 6 aromatic hydrocarbon rings through, for example, a direct-linkage, an alkylene group that may have an oxygen atom, a sulfur atom, a sulfonyl group, a carbonyl group and an alkylene group having carbonyloxy groups at the both ends thereof.

The divalent aromatic hydrocarbon groups indicated by R²' in the general formulae [1'] and [2'] include specifically, for example, divalent benzene, divalent naphthalene, divalent anthracene, divalent chrysene and a group represented by, for example, the following general formula [6']:

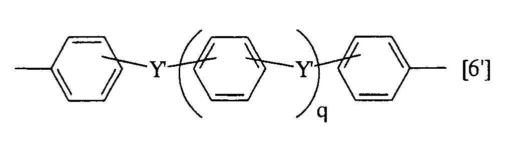

(wherein Y' indicates a direct-linkage, an alkylene group that may have an oxygen atom, a sulfur atom, a sulfonyl group, a carbonyl group and an alkylene group having carbonyloxy groups at the both ends thereof; q indicates an integer of 0 to 5). Said divalent aromatic hydrocarbon group may have further 1 to 10, preferably 1 to 5, more preferably 1 to 3 alkyl substituents in an aromatic ring portion thereof. Specific examples of such a group include the same as the alkyl substituents that the above tetravalent alicyclic hydrocarbon ring and aromatic hydrocarbon ring, indicated by R¹ and R¹' may have.

The above divalent aromatic hydrocarbon group indicated by R²' has at least 1 heavy hydrogen atom on the aromatic ring. Preferable are those having more hydrogen atoms of the aromatic ring thereof substituted by heavy hydrogen atoms, and those having more hydrogen atoms of the groups other than the aromatic ring substituted by heavy hydrogen atoms. More preferable are those having all hydrogen atoms on the aromatic ring thereof substituted by heavy hydrogen atoms. Particularly preferable are those having all hydrogen atoms of the divalent aromatic hydrocarbon group substituted by heavy hydrogen atoms.

The alkylene group of the_alkylene group that may have an oxygen atom indicated by Y' in the general formula [6] includes a group having usually 1 to 6, preferably 1 to 4, more preferably 1 to 2, and still more preferably 1 carbon atom. The alkylene group may be straight chained or branched, but preferably straight chained, and includes specifically, for example, a methylene group, an ethylene group, a n-propylene group, an isopropylene group, a n-butylene group, an isobutylene group, a sec-butylene group, a tert-butylene group, a n-pentylene group, an isopentylene group, a sec-pentylene group, a tert-pentylene group, a neopentylene group, a n-hexylene group, an isohexylene group, a sec-hexylene group and a tert-hexylene group. When having oxygen atoms, the alkylene group includes a group having usually 1 to 5, preferably 1 to 2, more preferably 1 oxygen atom at the end thereof or in the chain thereof.

The symbol q indicates an integer of usually 0 to 5, preferably 0 to 2, more preferably 0 or 1, and still more preferably 0.

Among deuterated polyimide compounds represented by the above general formula [1'], the deuterated polyimide compound represented by the following general formula [1"]:

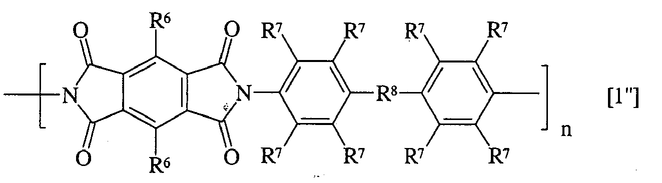

(wherein R⁶ s indicate each independently a heavy hydrogen atom or a light hydrogen atom; R⁷ s indicate each independently a heavy hydrogen atom or a deuterated methyl group; R⁸ indicates a direct-linkage or a deuterated methylene group; and n indicates an integer not less than 1, and provided that the partial structure: 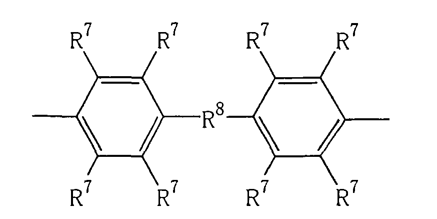 is deuterated) can be said to be industrially more useful compound from the standpoint of its performance as a raw material polymer for an optical waveguide. The deuterated polyamic acid compound represented by the general formula [2"]:

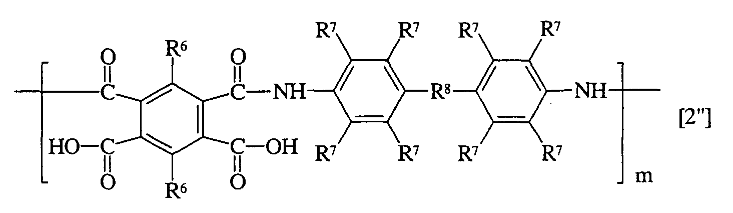

(wherein R⁶ s indicate each independently a heavy hydrogen atom or a light hydrogen atom; R⁷ s indicate each independently a heavy hydrogen atom or a deuterated methyl group; R⁸ indicates a direct-linkage or a deuterated methylene group; and m indicates an integer not less than 1, and provided that the partial structure: 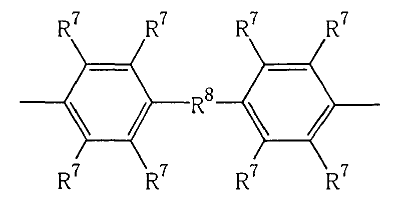 is deuterated) is an important intermediate to produce a compound represented by the above general formula [1"].

The acid anhydride represented by the general formula [3] that is used in the production method of the deuterated polyamic acid compound and deuterated polyimide compound of the present invention may be obtained on the market or synthesized by a known method where a suitable carboxylic acid is subjected to the action of a dehydrating agent or a condensing agent as appropriate. An acid anhydride on the market deuterated by a conventional method or an acid anhydride deuterated in advance, for example, according to a deuteration method of a diamine compound to be described later may be used.

The deuterated diamine compound represented by the general formula [4] that is used in the production method of the deuterated polyamic acid compound and deuterated polyimide compound of the present invention can be obtained by reacting, for example, the corresponding light hydrogen diamine compound with a heavy hydrogen source in the presence of a catalyst selected from an activated platinum catalyst, palladium catalyst, rhodium catalyst, ruthenium catalyst, nickel catalyst and cobalt catalyst.

The heavy hydrogen source to be used for deuteration of a diamine compound includes, a heavy hydrogen gas (D₂, T₂) and a deuterated solvent. In the case where the heavy hydrogen is a deuterium, the deuterated solvent includes, for example, deuterated water (D₂O), deuterated alcohols such as deuterated methanol, deuterated ethanol, deuterated isopropanol, deuterated butanol, deuterated tert-butanol, deuterated pentanol, deuterated hexanol, deuterated heptanol, deuterated octanol, deuterated nonanol, deuterated decanol, deuterated undecanol and deuterated dodecanol; deuterated carboxylic acids such as deuterated formic acid, deuterated acetic acid, deuterated propionic acid, deuterated butyric acid, deuterated isobutyric acid, deuterated valeric acid, deuterated isovaleric acid and deuterated pivalic acid; deuterated ketones such as deuterated acetone, deuterated methyl ethyl ketone, deuterated methyl isobutyl ketone, deuterated diethyl ketone, deuterated dipropyl ketone, deuterated diisopropyl ketone and deuterated dibutyl ketone; and organic solvents such as deuterated dimethyl sulfoxide. Among these, deuterated water and deuterated alcohols are preferable, and specifically deuterated water and deuterated methanol are more preferable. Deuterated water is particularly preferable in view of ecology and operability. In the case where the heavy hydrogen is a tritium, the deuterated solvent includes, for example, tritiated water (T₂O).

A deuterated solvent having at least 1 hydrogen atom in the molecule deuterated is useful. For example, deuterated alcohols having the hydrogen atom of the hydroxyl group deuterated and deuterated carboxylic acids having the hydrogen atom of the carboxyl group deuterated can be used for the deuteration method of diamine compounds. However, a solvent having all hydrogen atoms in the molecule deuterated is particularly preferable.

The more deuteration source is used, the further deuteration of a diamine compound proceeds. Considering an economical aspect, however, the lower limit of the amount of the heavy hydrogen atom contained in a heavy hydrogen source is preferable in the order of equimole, 10 molar times, 20 molar times, 30 molar times and 40 molar times, whereas the upper limit of the amount is preferable in the order of 250 molar times and 150 molar times, based on the amount of the deuteratable hydrogen atom in a substrate, that is, a light hydrogen diamine compound.

A reaction solvent may be used as necessary in deuteration of a diamine compound relating to the present invention. In the case of a liquid diamine compound as a reaction substrate, a reaction solvent is not necessary to use even when a heavy hydrogen gas is used as a heavy hydrogen source. When a deuterated solvent is used as a heavy hydrogen source, a reaction solvent is not necessary to use even in the case of a solid diamine compound as a reaction substrate. However, an appropriate reaction solvent is necessary to use when a reaction substrate is solid and a heavy hydrogen source is a heavy hydrogen gas.

Because a reaction system to deuterate a diamine compound may be in a suspended state, a solvent that hardly dissolves the diamine compound can be used as a reaction solvent to be used as necessary, but a solvent that easily dissolves a diamine compound is preferable. The specific example of the reaction solvent includes organic solvents which are not deuterated by a heavy hydrogen gas, comprising ethers such as dimethyl ether, diethyl ether, diisopropyl ether, ethylmethyl ether, tert-butylmethyl ether, 1,2-dimethoxyethane, oxirane, 1,4-dioxane, dihydropyrane and tetrahydrofuran; aliphatic hydrocarbons such as hexane, heptane, octane, nonane, decane and cyclohexane; and organic solvents which can be used as a heavy hydrogen source of the present invention even if deuterated by a heavy hydrogen gas, comprising alcohols such as methanol, ethanol, isopropanol, butanol, tert-butanol, pentanol, hexanol, heptanol, octanol, nonanol, decanol, undecanol and dodecanol; carboxylic acids such as formic acid, acetic acid, propionic acid, butyric acid, isobutyric acid, valeric acid, isovaleric acid and pivalic acid; ketones such as acetone, methyl ethyl ketone, methyl isobutyl ketone, diethyl ketone, dipropyl ketone, diisopropyl ketone and dibutyl ketone; and dimethylsulfoxide.

The catalyst to be used in deuteration of a diamine compound relating to the present invention, which is selected from an activated platinum catalyst, palladium catalyst, rhodium catalyst, ruthenium catalyst, nickel catalyst and cobalt catalyst (hereinafter may be abbreviated as an "activated catalyst") refers to a catalyst activated by bringing the so-called platinum catalyst, palladium catalyst, rhodium catalyst, ruthenium catalyst, nickel catalyst or cobalt catalyst (hereinafter may be abbreviated as a "non-activated catalyst" or simply a "catalyst") into contact with hydrogen gas or heavy hydrogen gas.

Deuteration of a diamine compound relating to the present invention may be carried out using a catalyst activated in advance. Activation of a catalyst and deuteration of a substrate may be carried out at the same time under coexistence of the non-activated catalyst and hydrogen gas or heavy hydrogen gas in the deuteration reaction system.

When deuteration is carried out using a catalyst activated in advance by hydrogen gas or heavy hydrogen gas, a gas phase in a deuteration reactor may be replaced by an inert gas such as nitrogen and argon.

To carry out a deuteration reaction of the present invention in the presence of hydrogen gas or heavy hydrogen gas in the reaction system, the hydrogen gas or heavy hydrogen gas may be passed directly through a reaction solution, or the gas phase in a deuteration reactor may be replaced by the hydrogen gas or heavy hydrogen gas.

In deuteration of a diamine compound relating to the present invention, a reactor is preferably under a sealed or a nearly-sealed condition resulting in a pressurized condition of the reaction system. The nearly-sealed condition is applied to the reaction such as the so-called continuous reaction where a reaction substrate is continuously injected to a reactor and a reaction product is continuously taken out of the reactor.

When a reactor is under a sealed condition in deuteration of a diamine compound relating to the present invention, the reaction temperature can be easily raised leading to efficient deuteration.

The catalyst to be used for deuteration of a diamine compound relating to the present invention includes a platinum catalyst, a palladium catalyst, a rhodium catalyst, a ruthenium catalyst, a nickel catalyst and a cobalt catalyst as mentioned above, and among these, a palladium catalyst, a platinum catalyst and a rhodium catalyst are preferable, with a palladium catalyst and a platinum catalyst more preferable and a platinum catalyst particularly preferable. Each of these catalysts can be effectively used for deuteration of a diamine compound by itself or in combination with one another, when it is activated by hydrogen gas or heavy hydrogen gas in a manner as mentioned above.

The palladium catalyst includes one having usually 0 to 4, preferably 0 to 2 and more preferably 0 valence of a palladium atom.

The platinum catalyst includes one having usually 0 to 4, preferably 0 to 2 and more preferably 0 valence of a platinum atom.

The rhodium catalyst includes one having usually 0 or 1, preferably 0 valence of a rhodium atom.

The ruthenium catalyst includes one having usually 0 to 2, preferably 0 valence of a ruthenium atom.

The nickel catalyst includes one having usually 0 to 2, preferably 0 valence of a nickel atom.

The cobalt catalyst includes one having usually 0 or 1, preferably 1 valence of a cobalt atom.

The above catalyst may be a metal itself, a metal oxide, a metal halide, a metal acetate or a metal having a ligand, or may be a metal itself, a metal oxide, a metal halide, a metal acetate or a metal complex, that is supported on various carriers.

Hereinafter, a catalyst supported on a carrier may be abbreviated as a "carrier-supported metal catalyst", and a catalyst not supported on a carrier may be abbreviated as a "metal catalyst".

The ligand of a metal catalyst which may have a ligand among the catalysts to be used for deuteration of a diamine compound relating to the present invention includes, for example, 1,5-cyclooctadiene (COD), dibenzylidene acetone (DBA), bipyridine (BPY), phenanthroline (PHE), benzonitrile (PhCN), isocyanide (RNC), triethylarsine (As(Et)₃), acetylacetonate (acac); an organic phosphine ligand such as dimethylphenylphosphine (P(CH₃)₂Ph), diphenylphosphinoferrocene (DPPF), trimethylphosphine (P(CH₃)₃), triethylphosphine (PEt₃), tri-tert-butylphosphine (P^{t}Bu₃), tricyclohexylphosphine (PCy₃), trimethoxyphosphine (P(OCH₃)₃), triethoxyphosphine (P(OEt)₃), tri-tert-butoxyphosphine (P(O^{t}Bu)₃), triphenylphosphine (PPh₃), 1,2-bis(diphenylphosphino)ethane (DPPE), triphenoxyphosphine (P(OPh)₃) and o-tolylphosphine (P(o-tolyl)₃).

Specific examples of the platinum based metal catalyst include, for example, Pt; platinum catalysts such as PtO₂, PtCl₄, PtCl₂ and K₂PtCl₄; platinum catalysts which are coordinated with a ligand such as PtCl₂(cod), PtCl₂(dba), PtCl₂(PCy₃)₂, PtCl₂(P(OEt)₃)₂, PtCl₂(P(O^{t}Bu)₃)₂, PtCl₂(bpy), PtCl₂(phe), Pt(PPh₃)₄, Pt(cod)₂, Pt(dba)₂, Pt(bpy)₂ and Pt(phe)₂.

Specific examples of the palladium based metal catalyst include, for example, Pd; palladium hydroxide catalysts such as Pd(OH)₂; palladium oxide catalysts such as PdO; halogenated palladium catalysts such as PdBr₂, PdCl₂ and PdI₂; palladium acetate catalysts such as palladium acetate (Pd(OAc)2) and palladium trifluoroacetate (Pd(OCOCF₃)₂); palladium metal complex catalysts which are coordinated with a ligand such as Pd(RNC)₂Cl₂, Pd(acac)₂, diacetate-bis-(triphenylphosphine)palladium [Pd(OAc)₂(PPh₃)₂], Pd(PPh₃)₄, Pd₂(dba)₃, Pd(NH₃)₂Cl₂, Pd(CH₃CN)₂Cl₂, dichloro-bis-(benzdnitrile)palladium [Pd(PhCN)₂Cl₂], Pd(dppe)Cl₂, Pd(dppf)Cl₂, Pd(PCy₃)₂Cl₂, Pd(PPh₃)₂Cl₂, Pd[P(o-tolyl)₃]₂Cl₂, Pd(cod)₂Cl₂ and Pd(PPh₃)(CH₃CN)₂Cl₂.

Specific examples of the rhodium based metal catalyst include, for example, Rh and rhodium catalysts which are coordinated with a ligand such as RhCl(PPh₃)₃.

Specific examples of the ruthenium based metal catalyst include, for example, Ru and ruthenium catalysts which are coordinated with a ligand such as RuCl₂(PPh₃)₃.

Specific examples of the nickel based metal catalyst include, for example, Ni; nickel catalysts such as NiCl₂ and NiO; nickel catalysts which are coordinated with a ligand such as NiCl₂(dppe), NiCl₂(PPh₃)₂, Ni(PPh₃)₄, Ni(P(OPh)₃)₄ and Ni(cod)₂.

Specific examples of the cobalt based metal catalyst include, for example, cobalt metal complex catalysts which are coordinated with a ligand such as Co(C₃H₅)[P(OCH₃)₃]₃.

The carrier, in the case where the above catalyst is supported on a carrier, includes, for example, carbon, alumina, silica gel, zeolite, molecular sieves, ion-exchange resins and polymers, and among these carbon is preferable.

The ion exchange resin used as a carrier may be a resin having no adverse effect on deuteration of a diamine compound, and includes, for example, a cation exchange resin and an anion exchange resin.

The cation exchange resin includes, for example, a weak acidic cation exchange resin and a strong acidic cation exchange resin. The anion exchange resin includes, for example, a weak basic anion exchange resin and a strong basic anion exchange resin.

The ion exchange resin generally contains a polymer cross-linked with a bifunctional monomer as a skeleton polymer, to which an acidic group or a basic group is bonded, and then is exchanged by various cations or anions (counter ions), respectively.

Specific examples of the weak acidic cation exchange resin include, for example, a resin obtained by hydrolysis of a polymer of an acrylic ester or a methacrylic ester cross-linked with divinylbenzene.

Specific examples of the strong acidic cation exchange resin include, for example, a resin obtained by sulfonation of a styrene-divinylbenzene copolymer.

Specific examples of the strong basic anion exchange resin include, for example, a resin obtained by bonding an amino group to an aromatic ring of a styrene-divinylbenzene copolymer.

Strength of basicity of a basic anion exchange resin increases with an amino group of in the order of a primary amino group, a secondary amino group, a tertiary amino group and a quaternary ammonium salt.

An ion exchange resin generally available on the market as well as the above ion exchange resin may be used as a carrier of a catalyst to be used for deuteration of the present invention.

The polymer used as a carrier is not especially limited unless it has an adverse effect on deuteration of a diamine compound, however, an example of such a polymer includes one obtained by polymerization or copolymerization of a monomer shown by the following general formula [7]:

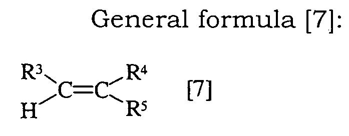

(wherein R³ indicates a hydrogen atom, a lower alkyl group, a carboxyl group, a carboxyalkyl group, an alkoxycarbonyl group, a hydroxyalkoxycarbonyl group, a cyano group or a formyl group; R⁴ indicates a hydrogen atom, a lower alkyl group, a carboxyl group, an alkoxycarbonyl group, a hydroxyalkoxycarbonyl group, a cyano group or a halogen atom; R⁵ indicates a hydrogen atom, a lower alkyl group, a haloalkyl group, a hydroxyl group, an aryl group which may have a substituent, an aliphatic heterocyclic group, an aromatic heterocyclic group, a halogen atom, an alkoxycarbonyl group, a hydroxyalkoxycarbonyl group, a sulfo group, a cyano group, a cyano-containing alkyl group, an acyloxy group, a carboxyl group, a carboxyalkyl group, an aldehyde group, an amino group, an aminoalkyl group, a carbamoyl group, an N-alkylcarbamoyl group or a hydroxyalkyl group; and R³ and R⁴ may form an alicyclic ring together with the adjacent -C=C- bond).

In general formula [7], the lower alkyl group indicated by R³ to R⁵ may be straight chained, branched or cyclic, and includes, for example, an alkyl group having 1 to 6 carbon atoms, which are specifically exemplified by, a methyl group, an ethyl group, a n-propyl group, an isopropyl group, a n-butyl group, an isobutyl group, a sec-butyl group, a tert-butyl group, a n-pentyl group, an isopentyl group, a sec-pentyl group, a tert-pentyl group, a 1-methylpentyl group, a neopentyl group, a n-hexyl group, an isohexyl group, a sec-hexyl group, a tert-hexyl group, a neohexyl group, a cyclopropyl group, a cyclopentyl group and a cyclohexyl group.

The carboxyalkyl group indicated by R³ and R⁵ includes, for example, one wherein a part of hydrogen atoms of the above lower alkyl group are replaced by a carboxyl group, and which are specifically exemplified by, for example, a carboxymethyl group, a carboxyethyl group, a carboxypropyl group, a carboxybutyl group, a carboxypentyl group and a carboxyhexyl group.

The alkoxycarbonyl group indicated by R³ to R⁵ includes preferably, for example, one having 2 to 11 carbon atoms and specifically, for example, a methoxycarbonyl group, an ethoxycarbonyl group, a propoxycarbonyl group, a butoxycarbonyl group, a pentyloxycarbonyl group, a hexyloxycarbonyl group, a heptyloxycarbonyl group, an 2-ethylhexyloxycarbonyl group, an octyloxycarbonyl group, a nonyloxycarbonyl group and a decyloxycarbonyl group.

The hydroxyalkoxycarbonyl group indicated by R³ to R⁵ includes one, wherein a part of hydrogen atoms of the above alkoxycarbonyl group having 2 to 11 carbon atoms are replaced by a hydroxyl group, which are specifically exemplified by, for example, a hydroxymethoxycarbonyl group, a hydroxyethoxycarbonyl group, a hydroxypropoxycarbonyl group, a hydroxybutoxycarbonyl group, a hydroxypentyloxycarbonyl group, a hydroxyhexyloxycarbonyl group, a hydroxyheptyloxycarbonyl group, a hydroxyoctyloxycarbonyl group, a hydroxynonyloxycarbonyl group and a hydroxydecyloxycarbonyl group.

The halogen atom indicated by R⁴ and R⁵ includes, for example, fluorine, chlorine, bromine and iodine.

The haloalkyl group indicated by R⁵ includes, for example, a group having 1 to 6 carbon atoms that is formed by halogenating (e.g., fluorinating, chlorinating, brominating and iodinating) the above lower alkyl group of 1 to 6 carbon atoms indicated by R³ to R⁵, which are specifically exemplified by, for example, a chloromethyl group, a bromomethyl group, a trifluoromethyl group, a 2-chloroethyl group, a 3-chloropropyl group, a 3-bromopropyl group, a 3,3,3-trifluoropropyl group, a 4-chlorobutyl group, a 5-chloropentyl group and a 6-chlorohexyl group.

The aryl group of the aryl group which may have a substituent includes a group having 6 to 10 carbon atoms, which are specifically exemplified by, for example, a phenyl group, a tolyl group, a xylyl group and a naphthyl group, and said substituent includes, for example, an amino group, a hydroxyl group, a lower alkoxy group having 1 to 6 carbon atoms and a carboxyl group. Specific examples of the substituted aryl group include, for example, an aminophenyl group, a toluidino group, a hydroxyphenyl group, a methoxyphenyl group, a tert-butoxyphenyl group and a carboxyphenyl group.

The aliphatic heterocyclic group includes, for example, a 5- or 6-membered ring having 1 to 3 hetero atoms such as a nitrogen atom, an oxygen atom and a sulfur atom, and specifically, for example, a pyrrolidyl-2-one group, a piperidyl group, a piperidino group, a piperazinyl group and a morpholino group.

The aromatic heterocyclic group includes, for example, a 5- or 6-membered ring having 1 to 3 hetero atoms such as a nitrogen atom, an oxygen atom and a sulfur atom, and specifically, for example, a pyridyl group, an imidazolyl group, a thiazolyl group, a furanyl group and a pyranyl group.

The cyano-containing alkyl group includes, for example, a group formed by replacing part of hydrogen atoms of the above lower alkyl group having 1 to 6 carbon atoms by cyano groups, and specifically, for example, a cyanomethyl group, a 2-cyanoethyl group, a 2-cyanopropyl group, a 3-cyanopropyl group, a 2-cyanobutyl group, a 4-cyanobutyl group, a 5-cyanopentyl group and a 6-cyanohexyl group.

The acyloxy group includes, for example, a group derived from a carboxylic acid having 2 to 20 carbon atoms and which are specifically exemplified by, for example, an acetyloxy group, a propionyloxy group, a butyryloxy group, a pentanoyloxy group, a nonanoyloxy group, a decanoyloxy group and a benzoyloxy group.

The aminoalkyl group includes a group formed by replacing part of hydrogen atoms of the above lower alkyl group having 1 to 6 carbon atoms by amino groups, and specifically, for example, an aminomethyl group, an aminoethyl group, an aminopropyl group, an aminobutyl group, an aminopentyl group and an aminohexyl group.

The N-alkylcarbamoyl group includes a group formed by replacing part of hydrogen atoms of a carbamoyl group by the above lower alkyl group having 1 to 6 carbon atoms, and specifically, for example, an N-methylcarbamoyl group, an N-ethylcarbamoyl group, an N-n-propylcarbamoyl group, an N-isopropylcarbamoyl group, an N-n-butylcarbamoyl group and an N-tert-butylcarbamoyl group.

The hydroxyalkyl group includes a group formed by replacing part of hydrogen atoms of the above lower alkyl group having 1 to 6 carbon atoms by hydroxyl groups, and specifically, for example, a hydroxymethyl group, a hydroxyethyl group, a hydroxypropyl group, a hydroxybutyl group, a hydroxypentyl group and a hydroxyhexyl group.

The alicyclic ring in the case where R³ and R⁴ are bonded together with the adjacent -C=C- group to form an alicyclic ring, may be monocyclic or polycyclic and includes, for example, an unsaturated alicyclic ring having 5 to 10 carbon atoms, and specifically, for example, a norbornene ring, a cyclopentene ring, a cyclohexene ring, a cyclooctene ring and a cyclodecene ring.

The specific examples of the monomer represented by the general formula [7] include ethylenically unsaturated aliphatic hydrocarbons having 2 to 20 carbon atoms such as ethylene, propylene, butylene and isobutylene; ethylenically unsaturated aromatic hydrocarbons having 8 to 20 carbon atoms such as styrene, 4-methylstyrene, 4-ethylstyrene and divinylbenzene; alkenyl esters having 3 to 20 carbon atoms such as vinyl formate, vinyl acetate, vinyl propionate and isopropenyl acetate; halogen-containing ethylenically unsaturated compounds having 2 to 20 carbon atoms such as vinyl chloride, vinylidene chloride, vinylidene fluoride and tetrafluoroethylene; ethylenically unsaturated carboxylic acids having 3 to 20 carbon atoms such as acrylic acid, methacrylic acid, itaconic acid, maleic acid, fumaric acid, crotonic acid, vinylacetic acid, allylacetic acid and vinylbenzoic acid (These acids may take a form of a salt of an alkali metal such as sodium and potassium or an ammonium salt.); ethylenically unsaturated carboxylic esters such as methyl methacrylate, ethyl methacrylate, propyl methacrylate, butyl methacrylate, 2-ethylhexyl methacrylate, methyl acrylate, ethyl acrylate, propyl acrylate, butyl acrylate, 2-ethylhexyl acrylate, lauryl methacrylate, stearyl acrylate, methyl itaconate, ethyl itaconate, methyl maleate, ethyl maleate, methyl fumarate, ethyl fumarate, methyl crotonate, ethyl crotonate and methyl 3-butenoate; cyano-containing ethylenically unsaturated compounds having 3 to 20 carbon atoms such as acrylonitrile, methacrylonitrile and allyl cyanide; ethylenically unsaturated amide compounds having 3 to 20 carbon atoms such as acrylamide and methacrylamide; ethylenically unsaturated aldehydes having 3 to 20 carbon atoms such as acrolein and crotonaldehyde; ethylenically unsaturated sulfonic acids having 2 to 20 carbon atoms such as vinylsulfonic acid and 4-vinylbenzene sulfonic acid (These acids may take a form of a salt of an alkali metal such as sodium and potassium.); ethylenically unsaturated aliphatic amines having 2 to 20 carbon atoms such as vinylamine and allylamine; ethylenicically unsaturated aromatic amines having 8 to 20 carbon atoms such as vinylaniline; ethylenically unsaturated aliphatic heterocyclic amines having 5 to 20 carbon atoms such as N-vinylpyrrolidone and vinylpiperidine; ethylenically unsaturated alcohols having 3 to 20 carbon atoms such as allyl alcohol and crotyl alcohol; ethylenically unsaturated phenols having 8 to 20 carbon atoms such as 4-vinylphenol and the like.

When the above polymers and the like are used as a carrier, a carrier that is hardly deuterated itself in deuteration of a diamine compound is preferably used. However, a catalyst supported on a deuteratable carrier itself can also be used for deuteration of the present invention.

In deuteration of a light hydrogen diamine compound relating to the present invention, a carrier-supported palladium catalyst, a carrier-supported platinum catalyst or a carrier-supported rhodium catalyst is preferably used among various carrier-supported catalysts, and a carrier-supported palladium catalyst, a carrier-supported platinum catalyst and a mixture thereof are more preferably used.

In the carrier-supported catalyst, content of the catalyst metal, which is palladium, platinum, rhodium, ruthenium, nickel or cobalt, is usually 1 to 99% by weight, preferably 1 to 50% by weight, more preferably 1 to 30% by weight, still more preferably 1 to 20% by weight, and particularly preferably 1 to 10% by weight, based on the total amount of the catalyst.

In deuteration of a light hydrogen diamine compound relating to the present invention, the amount of an activated catalyst or a non-activated catalyst to be used is usually a so-called catalyst amount, preferably in the order of, 0.01 to 200% by weight, 0.01 to 100% by weight, 0.01 to 50% by weight, 0.01 to 20% by weight, 0.1 to 20% by weight, 1 to 20% by weight and 10 to 20% by weight, based on a light hydrogen diamine compound to be used as a reaction substrate regardless of whether the catalyst is supported by a carrier or not, and the upper limit content of the catalyst metal in said whole catalyst is preferably in the order of, 20% by weight, 10% by weight, 5% by weight and 2% by weight, whereas the lower limit content is preferably in the order of, 0.0005% by weight, 0.005% by weight, 0.05% by weight and 0.5% by weight.

In deuteration of a diamine compound, a combined catalyst of 2 or more catalysts among the above various catalysts can be used and sometimes serves to improve a deuteration ratio. Such a combination of catalysts includes, for example, a combination of a palladium catalyst and a platinum catalyst, a ruthenium catalyst or a rhodium catalyst, a combination of a platinum catalyst and a ruthenium catalyst or a rhodium catalyst, and a combination of a ruthenium catalyst and a rhodium catalyst. Among these combinations, a combination of a palladium catalyst and a platinum catalyst is preferable, and one or both of them may be supported by a carrier. Preferable specific examples include a combination of a palladium carbon and a platinum carbon.

The amount of the catalysts to be used in combination of 2 or more catalysts may be set so that the total amount of the catalysts in the combination may become the above mentioned amount of a catalyst. The ratio of the amount of each catalyst to be used is not particularly limited. For example, in the case where the above combination of a palladium carbon and a platinum carbon, the weight of palladium in the catalyst may be set usually 0.01 to 100 times, preferably 0.1 to 10 times, more preferably 0.2 to 5 times, relative to the weight of platinum.

In the case where a non-activated catalyst is used in deuteration of a diamine compound, the amount of hydrogen to be used when the hydrogen is present in the reaction system to activate a non-activated catalyst may be the necessary amount to activate the catalyst, usually 1 to 20,000 equivalents and preferably 10 to 700 equivalents, based on the catalyst, because an excessive amount of hydrogen hydrogenates a deuterated solvent served as a heavy hydrogen source or lowers a ratio of heavy hydrogen served as a heavy hydrogen source in the reaction system have an adverse effect on the deuteration reaction of a diamine compound.

In the case where heavy hydrogen is used to activate a catalyst in the reaction system, the amount of the heavy hydrogen to be used when the hydrogen is present may be enough to activate the catalyst and usually 1 to 20,000 equivalents and preferably 10 to 700 equivalents, based on the catalyst. However, because said heavy hydrogen can be used also as a heavy hydrogen source, an excessive amount of the heavy hydrogen can carry out deuteration of a diamine compound without any problem.

With regard to reaction temperature in deuteration of a light hydrogen diamine compound relating to the present invention, the lower limit is usually 10°C, preferably in the order of 20°C, 40°C, 60°C, 80°C, 110°C, 140°C and 160°C, whereas the upper limit is usually 300°C, preferably in the order of 200°C and 180°C.

Reaction time for deuteration is usually 30 minutes to 72 hours, preferably 1 to 48 hours, more preferably 3 to 30 hours and still more preferably 6 to 24 hours.

Deuteration of a light hydrogen diamine compound relating to the present invention is specifically described by taking as an example the case to use a heavy water as a heavy hydrogen source and use a mixed catalyst of a palladium carbon (Pd/C) (Pd content: 10%) and a platinum carbon (Pt/ C) (Pt content: 5%) as a non-activated catalyst.

For example, 1 mole of a light hydrogen diamine compound (substrate) corresponding to a deuterated diamine compound represented by the general formula [4] relating to the present invention and a mixed catalyst composed of 0.1 to 1% by weight of Pd/C based on the substrate that is activated in advance in contact with hydrogen gas and 0.1 to 1% by weight of Pt/C based on the substrate that is activated in advance in contact with hydrogen gas are added to deuterated water the amount of which is enough to contain 10 to 150 molar times of heavy hydrogen atoms based on the deuteratable hydrogen atoms of the substrate. The reactor is sealed and has its gas phase replaced by an inert gas. Reaction is carried out at about 110 to 200°C in an oil bath for about 1 to 48 hours under stirring, to easily obtain the deuterated diamine compound represented by the general formula [4].

The amount of the heavy hydrogen atoms that are contained in an aromatic hydrocarbon group indicated by R² in the obtained deuterated diamine compound represented by the general formula [4] is usually 20% or more, preferably 20 to 100%, more preferably 40 to 100%, still more preferably 60 to 100% and further still more preferably 80 to 100%, based on the amount of the hydrogen atoms that are contained in the aromatic hydrocarbon group.

In the method for producing a deuterated polyamic acid compound of the present invention, it is desirable that the above reaction of an acid anhydride represented by the general formula [3] and a deuterated diamine compound represented by the general formula [4] be carried out in a suitable solvent.

The solvent to be used may be a polar solvent dissolving an acid anhydride and a deuterated diamine compound, which is specifically exemplified by an amide-based solvent such as dimethylacetamide, N-methylpyrrolidone and dimethylformamide; a sulfoxide-based solvent such as dimethyl sulfoxide and diethyl sulfoxide; and a phenol-based solvent such as phenol and o-, m- and p-cresols.

The reaction temperature of an acid anhydride and a deuterated diamine compound in the production method of the present invention is usually 0 to 50°C, preferably 10 to 40°C and more preferably 15 to 35°C.

The reaction time of an acid anhydride and a deuterated diamine compound is usually 0.1 to 5 hours, preferably 0.5 to 3 hours and more preferably 1 to 2 hours.

A deuterated polyimide compound represented by the general formula [1] relating to the present invention can be easily obtained by a ring closure reaction (hereinafter, may be abbreviated as "cyclization step") of a deuterated polyamic acid compound represented by the general formula [2]. The deuterated polyamic acid compound to be used is preferably obtained by the above mentioned method.

The above ring closure reaction may be usually carried out by a ring closure reaction in this field and includes specifically, for example, a cyclization under heating and a chemical cyclization in the presence of a basic catalyst and a dehydrating agent.

In the case where a deuterated polyamic acid compound is subjected to a ring closure reaction under heating as mentioned above, the reaction temperature is usually 150 to 500°C, preferably 250 to 400°C and more preferably 250 to 350°C, and the reaction time is usually 0.1 to 10 hours, preferably 1 to 5 hours and more preferably 1 to 2 hours.

In the case where a deuterated polyamic acid compound is subjected to a chemical cyclization in the presence of a basic catalyst and a dehydrating agent as mentioned above, the basic catalyst to be used includes, for example, pyridine, triethylamine and quinoline, and the amount to be used is usually 3 to 30 molar times based on 1 mole of the repeating unit of the deuterated polyamic acid compound.

The dehydrating agent to be used in a chemical cyclization includes, for example, acetic anhydride, propionic anhydride and trifluoroacetic anhydride, and the amount to be used is 1.5 to 20 molar times based on 1 mole of the repeating unit of the polyamic acid compound.

The reaction temperature in a chemical cyclization in the presence of a basic catalyst and a dehydrating agent is usually 20 to 200°C.

As a deuterated polyamic acid compound to be used in the above cyclization step, it is also possible to use a reaction solution as it is, which is one containing the deuterated polyamic acid compound (reaction intermediate) represented by the general formula [2], obtained after reacting an acid anhydride represented by the general formula [3] and a deuterated diamine compound represented by the general formula [4] in the similar way to the above production method of a deuterated polyamic acid compound.

A deuterated polyimide compound represented by the general formula [1], obtained by the production method of the present invention has an extremely higher deuteration ratio compared with conventional polyimide compounds and therefore is useful as a raw material of a polymer for an optical waveguide that has excellent transparency and heat resistance, low moisture absorption, a small optical transmission loss, a high refractive index and good adhesion to a base material or a substrate.

A deuterated polyimide compound having a deuteration ratio of usually 20 to 100%, preferably 40 to 100%, more preferably 60 to 100% and still more preferably 80 to 100%, based on the total deuteratable hydrogen atoms contained in the deuterated polyimide compound represented by the general formula [1] shows the above properties remarkably and thus is particularly desirable as a raw material of a polymer for an optical waveguide. In particular, a deuterated polyimide compound containing a divalent aromatic hydrocarbon group having a heavy hydrogen atom indicated by R² in the general formula [1] that has a ratio of heavy hydrogen atoms of usually 20% or more, preferably 40% or more, more preferably 60% or more and still more preferably 80% or more, based on the hydrogen atoms contained therein is useful as a raw material polymer for an optical waveguide.

It can be said that a deuterated polyamic acid compound represented by the general formula [2], obtained by the production method of the present invention is an important reaction intermediate or raw material for producing a deuterated polyimide compound useful as a raw material of a polymer for an optical waveguide.

When a deuterated polyimide compound represented by the general formula [1], obtained by the production method of the present invention is used as a raw material polymer for an optical waveguide, the deuterated polyimide compound is preferably used as a film.

A method for film formation of a deuterated polyimide compound is not limited as long as it is based on a conventional film formation step in this field using the obtained deuterated polyimide compound, and includes, for example, a method of carrying out a cyclization step for producing a deuterated polyimide compound from a deuterated polyamic acid compound and a film formation step of the obtained deuterated polyimide compound at the same time. Specifically, for example, a deuterated polyamic acid compound represented by the general formula [2] of the present invention is cast on a glass Petri dish or the like and then subjected to a ring closure reaction under heating to obtain a film of the deuterated polyimide compound directly from the deuterated polyamic acid compound as the intermediate.

When carrying out a step for cyclization and a step for film formation at the same time as mentioned above, a cyclization reaction is carried out under heating, and the heating temperature may be similar to the reaction temperature for cyclization of a deuterated polyamic acid compound as mentioned above. A rapid rise of reaction temperature is dangerous due to, for example, abrupt evaporation of a solvent such as an organic solvent, and also brings about a higher volume shrinkage factor through dehydration caused by the rapid ring closure reaction leading to foaming or cracking in the obtained film. However, it is desirable to carry out two-stage heating or to heat gradually from a room temperature through an evaporation temperature of a solvent up to a completion temperature of a ring closure reaction.

In two-stage heating, the temperature is not limited as long as it does not cause above problems. In the case where the solvent used in synthesizing a deuterated polyamic acid compound is contained in the compound, the initial temperature may be a temperature at which the solvent evaporates and is usually 40 to 250°C, preferably 100 to 250°C and more preferably 150 to 250°C. The next temperature may be a temperature at which the ring closure reaction proceeds and is usually 190 to 500°C, preferably 250 to 400°C and more preferably 250 to 350°C. The time of the ring closure reaction is usually 0.1 to 10 hours, preferably 1 to 5 hours and more preferably 1 to 2 hours.

When intending to form a film of a deuterated polyimide compound having good processability under more stable conditions without suffering a film loss or reduced film thickness, it is desirable to take the method of carrying out a ring closure reaction under heating (cyclization under heating) or a ring closure reaction in the presence of a basic catalyst and a dehydrating agent (chemical cyclization), depositing by an ordinary method the deuterated polyimide compound obtained in the above cyclization step and then dissolving the deposit in a suitable solvent, followed by applying and drying by an ordinary method such as a spin coating method and a casting method to form a film easily.

The method for film formation is specifically described in detail taking the spin coating method as an example. The method comprises dissolving an obtained deuterated polyimide compound in a suitable solvent, filtering the solution, applying the filtrate on a substrate such as a silicon wafer by spin coating and then heating at 100 to 250°C on a hot plate for 0.5 to 2 hours to form a film of the deuterated polyimide compound.

The solvent to be used in forming a film is not particularly limited as long as it dissolves the deuterated polyimide compound. The solvent includes specifically, for example, N-methyl-2-pyrrolidone (NMP), N,N-dimethylformamide (DMF) and dimethyl sulfoxide (DMSO).

The deuterated polyimide compound represented by the general formula [1], obtained by the production method of the present invention includes one preferably soluble in the solvent to be used for the film formation when subjected to film formation after depositing, and such deuterated polyimide compound is shown below.

The alicyclic hydrocarbon group in the tetravalent alicyclic hydrocarbon group indicated by R¹ in the general formula [1] that may have a heavy hydrogen atom is preferably a group derived from a polycyclic ring and includes specifically, for example, a monocyclic ring such as a cyclobutane ring, a cyclopentane ring, a cyclohexane ring, a cycloheptane ring, a cyclooctane ring, a cyclononane ring, a cyclodecane ring, a cycloundecane ring and a cyclododecane ring; a polycyclic ring formed by combining a few cross-linked rings such as a norbornane ring and a bicyclo[2.2.2]oct-2-ene ring at optional positions; and a tetravalent group derived from the above cross-linked rings and the like.

The aromatic hydrocarbon group in the tetravalent aromatic hydrocarbon group that may have a heavy hydrogen atom, indicated by R¹ is preferably a group represented by the general formula [5], more preferably a group where A in the general formula [5] is an oxygen atom, a carbonyl group, a direct-linkage or a sulfonyl group, still more preferably a group where A is an oxygen atom, a carbonyl group or a direct-linkage and further still more preferably a group where A is an oxygen atom.

The tetravalent aromatic hydrocarbon group has preferably an alkyl substituent, more preferably 1 to 4 alkyl substituents having 1 to 6 carbon atoms.

The symbol p in the general formula [5] is preferably an integer of 0 or 1.

The divalent aromatic hydrocarbon group indicated by R² in the general formula [1] is preferably a group represented by the general formula [6], more preferably a group where Y in the general formula [6] is a sulfonyl group, an oxygen atom, a carbonyl group or an alkylene group, still more preferably a group where Y is a sulfonyl group, an oxygen atom or a carbonyl group and further still more preferably a group where Y is a sulfonyl group.

The symbol q in the general formula [6] is preferably an integer of 0 or 1, more preferably an integer of 1.

The divalent aromatic hydrocarbon group has preferably an alkyl substituent, more preferably 1 to 4 alkyl substituents having 1 to 6 carbon atoms.

The present invention is described in more detail with reference to the following examples, to which, however, the present invention is not limited at all.

### Examples

### Reference Example 1. Synthesis of deuterated o-tolidine

o-Tolidine of 20 g and a mixed catalyst of 6 g composed of 10% Pd/ C of 2 g and 5% Pt/ C of 4 g were added to deuterated water (D₂O) of 680 mL and subjected to reaction at about 180°C for 24 hours. After termination of the reaction, the reaction solution was extracted with ethyl acetate, followed by filtering off the mixed catalyst. The obtained filtrate was dried using magnesium sulfate, concentrated under reduced pressure and then purified by column chromatography to obtain deuterated o-tolidine of 15.4 g (yield: 77%). The obtained deuterated o-tolidine was subjected to structural analysis by measuring its ¹H-NMR and ²H-NMR spectra to show an average deuteration ratio of 82%.

### Example 1. Synthesis of deuterated polyamic acid compound

Deuterated o-tolidine of 2.364 g (10 mmol) obtained in Reference Example 1 and pyromellitic dianhydride of 2.182 g (10 mmol) were added to dimethylacetamide of 41 g and subjected to reaction at about 25°C for about 2 hours, followed by ordinary processing to obtain 4.0 g of a deuterated polyamic acid compound having the following repeating constitution (weight average molecular weight: 168,000) (yield: 88%).

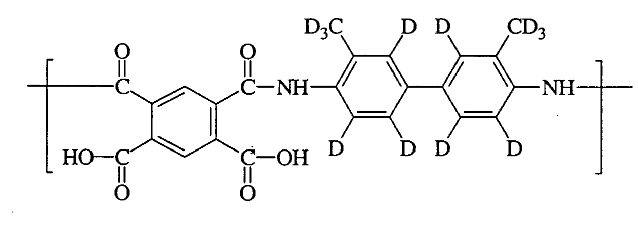

The IR spectrum of the obtained compound showed a small peak derived from C-H bonds in an aromatic ring around 3,000 cm⁻¹, which indicated an extremely small number of C-H bonds in the obtained compound. The obtained deuterated polyamic acid compound was subjected to structural analysis by measuring its ¹H-NMR and ²H-NMR spectra to show an average deuteration ratio of 70%.

### Example 2. Synthesis of deuterated polyimide compound

1 g of 10% by weight dimethylacetamide solution of the deuterated polyamic acid compound obtained in Example 1 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

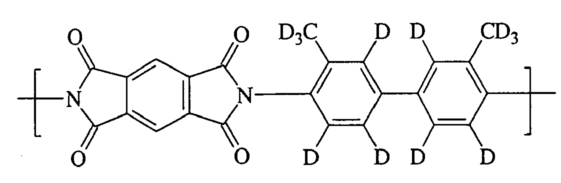

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Comparative Example 1.

o-Tolidine of 1.973 g (10 mmol) not having a heavy hydrogen atom and pyromellitic dianhydride of 2.027 g (10 mmol) were added to N-methylpyrrolidone of 36 g and subjected to reaction at about 25°C for about 2 hours, followed by ordinary processing to obtain a polyamic acid compound. 1 g of 10% by weight N-methylpyrrolidone solution of the polyamic acid compound was cast on a glass Petri dish, heated at about 200°C for about 1 hour and then subjected to reaction at about 300°C for about 1 hour to obtain 0.1 g of a polyimide compound (yield: 100%).

### Reference Example 2. Synthesis of deuterated 4,4'-methylene di-o-toluidine

4,4'-Methylene di-o-toluidine of 20 g (0.09 mol) and a mixed catalyst of 6 g composed of 10% Pd/ C of 2 g and 5% Pt/ C of 4 g were added to deuterated water (D₂O) of 680 mL and subjected to reaction at about 180°C for about 24 hours. After termination of the reaction, the reaction solution was extracted with ethyl acetate, followed by filtering off the mixed catalyst. The obtained filtrate was dried using magnesium sulfate, concentrated under reduced pressure and then purified by column chromatography to obtain deuterated 4,4'-methylene di-o-toluidine of 6.0 g (yield: 30%). The obtained deuterated 4,4'-methylene di-o-toluidine was subjected to structural analysis by measuring its ¹H-NMR and ²H-NMR spectra to show an average deuteration ratio of 81%.

### Example 3. Synthesis of deuterated polyamic acid compound

Deuterated 4,4'-methylene di-o-toluidine of 2.404 g (10 mmol) obtained in Reference Example 2 and pyromellitic dianhydride of 2.181 g (10 mmol) were added to N-methylpyrrolidone of 41 g and subjected to reaction at about 25°C for about 2 hours, followed by ordinary processing to obtain 4.0 g of a deuterated polyamic acid compound having the following repeating constitution (weight average molecular weight: 156,000) (yield: 87%).

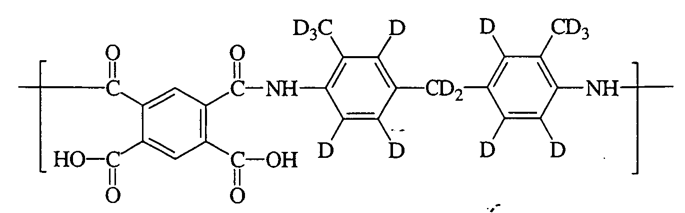

### Example 4. Synthesis of deuterated polyimide compound

1 g of 10% by weight N-methylpyrrolidone solution of the deuterated polyamic acid compound obtained in Example 3 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

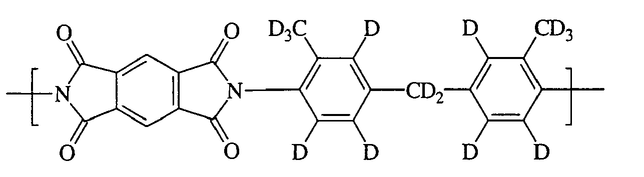

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Comparative Example 2.

A polyimide compound was obtained similarly as in Comparative Example 1 except that 10 mmol of 4,4'-methylene di-o-toluidine not having a heavy hydrogen atom was used instead of 10 mmol of o-tolidine not having a heavy hydrogen atom (yield: 98%).

### Reference Example 3. Synthesis of deuterated 3,3',5,5'-tetramethylbenzidine

3,3',5,5'-Tetramethylbenzidine of 10 g and a mixed catalyst of 3 g composed of 10% Pd/C of 1 g and 5% Pt/C of 2 g were added to deuterated water (D₂O) of 340 mL and subjected to reaction at 180°C for 24 hours. After termination of the reaction, the reaction solution was extracted with ethyl acetate, followed by filtering off the mixed catalyst. The obtained filtrate was dried using magnesium sulfate, concentrated under reduced pressure and then purified by column chromatography to obtain deuterated 3,3',5,5'-tetramethylbenzidine of 5.4 g (yield: 54%). The obtained deuterated 3,3',5,5'-tetramethylbenzidine was subjected to structural analysis by measuring its ¹H-NMR and ²H-NMR spectra to show an average deuteration ratio of 98%.

### Example 5. Synthesis of deuterated polyamic acid compound

Deuterated 3,3',5,5'-tetramethylbenzidine of 2.682 g (10 mmol) obtained in Reference Example 3 and pyromellitic dianhydride of 2.181 g (10 mmol) were added to N-methylpyrrolidone of 44 g and subjected to reaction at about 25°C for about 2 hours, followed by ordinary processing to obtain 3.9 g of a deuterated polyamic acid compound having the following repeating constitution (weight average molecular weight: 127,000) (yield: 80%).

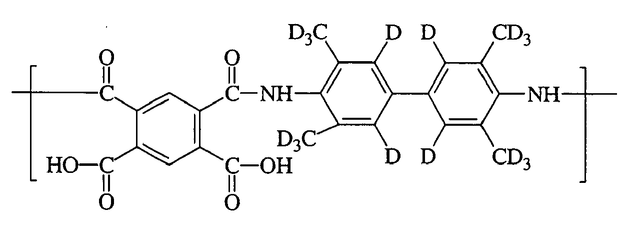

### Example 6. Synthesis of deuterated polyimide compound

1 g of 10% by weight N-methylpyrrolidone solution of the deuterated polyamic acid compound obtained in Example 5 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

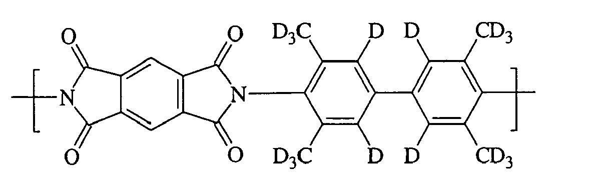

The obtained deuterated polyimide compound was subjected to structural analysis by measuring its IR spectrum, which indicated that the raw material deuterated polyamic acid compound was cyclized and the object deuterated polyimide compound was obtained.

### Comparative Example 3.

A polyimide compound was obtained similarly as in Comparative Example 1 except that 10 mmol of 3,3',5,5'-tetramethylbenzidine not having a heavy hydrogen atom was used instead of 10 mmol of o-tolidine not having a heavy hydrogen atom (yield: 96%).

### Reference Example 4. Synthesis of deuterated 4,4'-methylene di-2,6-xylidine

4,4'-Methylene di-2,6-xylidine of 10 g and a mixed catalyst of 3 g composed of 10% Pd/ C of 1 g and 5% Pt/ C of 2 g were added to deuterated water (D₂O) of 340 mL and subjected to reaction at about 180°C for about 24 hours. After termination of the reaction, the reaction solution was extracted with ethyl acetate, followed by filtering off the mixed catalyst. The obtained filtrate was dried using magnesium sulfate, concentrated under reduced pressure and then purified by column chromatography to obtain deuterated 4,4'-methylene di-2,6-xylidine of 6.3 g (yield: 63%). The obtained deuterated 4,4'-methylene di-2,6-xylidine was subjected to structural analysis by measuring its ¹H-NMR and ²H-NMR spectra to show an average deuteration ratio of 79%.

### Example 7. Synthesis of deuterated polyamic acid compound

Deuterated 4,4'-methylene di-2,6-xylidine of 2.724 g (10 mmol) obtained in Reference Example 4 and pyromellitic dianhydride of 2.181 g (10 mmol) were added to N-methylpyrrolidone of 44 g and subjected to reaction at about 25°C for about 2 hours, followed by ordinary processing to obtain 3.8 g of a deuterated polyamic acid compound having the following repeating constitution (weight average molecular weight: 134,000) (yield: 82%).

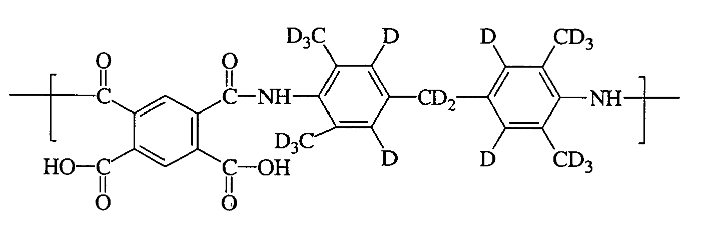

### Example 8. Synthesis of deuterated polyimide compound

1 g of 10% by weight N-methylpyrrolidone solution of the deuterated polyamic acid compound obtained in Example 7 was cast on a glass Petri dish, heated at about 200°C for about 1 hour and then subjected to reaction at about 300°C for about 1 hour to obtain 0.085 g of a deuterated polyimide compound having the following repeating constitution (yield: 85%).

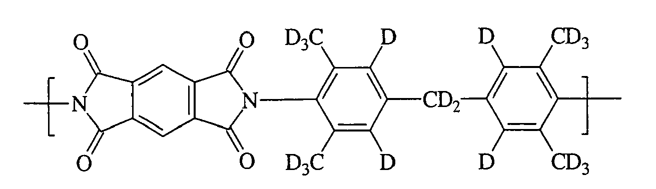

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Comparative Example 4.

A polyimide compound was obtained similarly as in Comparative Example 1 except that 10 mmol of 4,4'-methylene di-2,6-xylidine not having a heavy hydrogen atom was used instead of 10 mmol of o-tolidine not having a heavy hydrogen atom (yield: 98%).

### Reference Example 5. Synthesis of deuterated 4,4'-diaminodiphenylmethane

4,4'-Diaminodiphenylmethane of 10 g and a mixed catalyst of 3 g composed of 10% Pd/C of 1 g and 5% Pt/C of 2 g were added to deuterated water (D₂O) of 340 mL and subjected to reaction at about 180°C for 24 hours. After termination of the reaction, the reaction product was purified similarly as in Reference Example 1 to obtain deuterated 4,4'-diaminodiphenylmethane of 8 g (yield: 80%). The obtained deuterated 4,4'-diaminodiphenylmethane was subjected to structural analysis by measuring its ¹H-NMR and ²H-NMR spectra to show an average deuteration ratio of 96%.

### Example 9. Synthesis of deuterated polyamic acid compound

bDeuterated 4,4'-diaminodiphenylmethane of 10 mmol obtained in Reference Example 5 and pyromellitic dianhydride of 10 mmol were added to N-methylpyrrolidone of 36 g and subjected to reaction at about 25°C for about 2 hours, followed by ordinary processing to obtain 4 g of a deuterated polyamic acid compound having the following repeating constitution (weight average molecular weight: 145,000) (yield: 96%).

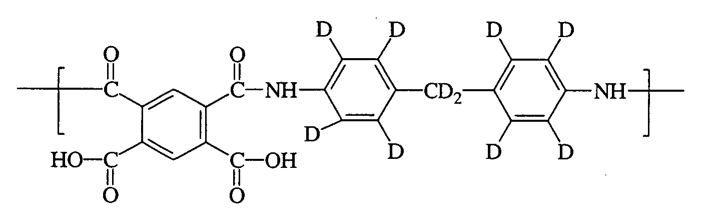

### Example 10. Synthesis of deuterated polyimide compound

1 g of 10% by weight N-methylpyrrolidone solution of the deuterated polyamic acid compound obtained in Example 9 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

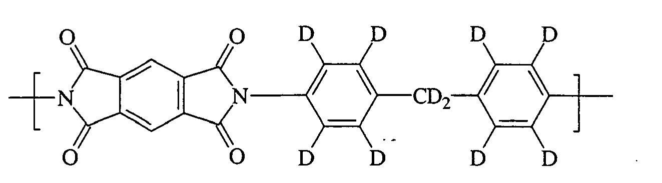

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Comparative Example 5.

A polyimide compound was obtained similarly as in Comparative Example 1 except that 10 mmol of 4,4'-diaminodiphenylmethane not having a heavy hydrogen atom was used instead of 10 mmol of o-tolidine not having a heavy hydrogen atom (yield: 95%).

### Reference Example 6. Synthesis of deuterated 4,4'-diaminodiphenyl ether

4,4'-Diaminodiphenyl ether of 10 g and a mixed catalyst of 3 g composed of 10% Pd/C of 1 g and 5% Pt/C of 2 g were added to deuterated water (D₂O) of 340 mL and subjected to reaction at about 180°C for 24 hours. After termination of the reaction, the reaction product was purified similarly as in Reference Example 1 to obtain deuterated 4,4'-diaminodiphenyl ether of 7.5 g (yield: 75%). The obtained deuterated 4,4'-diaminodiphenyl ether was subjected to structural analysis by measuring its ¹H-NMR and ²H-NMR spectra to show an average deuteration ratio of 98%.

### Example 11. Synthesis of deuterated polyamic acid compound

Deuterated 4,4'-diaminodiphenyl ether of 10 mmol obtained in Reference Example 6 and pyromellitic dianhydride of 10 mmol were added to N-methylpyrrolidone of 36 g and subjected to reaction at about 25°C for about 2 hours, followed by ordinary processing to obtain 4 g of a deuterated polyamic acid compound having the following repeating constitution (weight average molecular weight: 185,000) (yield: 100%).

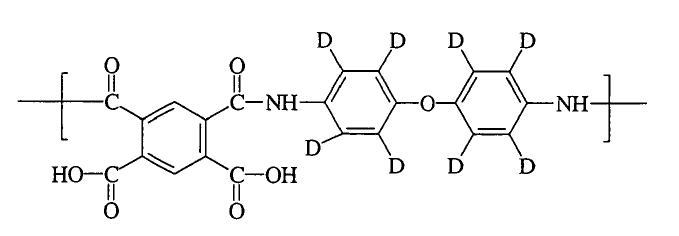

### Example 12. Synthesis of deuterated polyimide compound

1 g of 10% by weight N-methylpyrrolidone solution of the deuterated polyamic acid compound obtained in Example 11 was cast on a glass Petri dish, heated at about 200°C for about 1 hour and then subjected to reaction at about 300°C for about 1 hour to obtain 0.1 g of a deuterated polyimide compound having the following repeating constitution (yield: 100%).

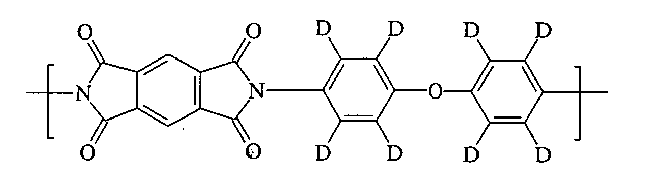

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Comparative Example 6.

A polyimide compound was obtained similarly as in Comparative Example 1 except for using 10 mmol of 4,4'-diaminodiphenyl ether not having a heavy hydrogen atom instead of 10 mmol of o-tolidine not having a heavy hydrogen atom (yield: 100%).

### Reference Example 7. Synthesis of deuterated 4,4'-diaminobenzophenone

4,4'-Diaminobenzophenone of 10 g and a mixed catalyst of 3 g composed of 10% Pd/ C of 1 g and 5% Pt/ C of 2 g were added to deuterated water (D₂O) of 340 mL and subjected to reaction at about 180°C for 24 hours. After termination of the reaction, the reaction product was purified similarly as in Reference Example 1 to obtain deuterated 4,4'-diaminobenzophenone of 9.6 g (yield: 96%). The obtained deuterated 4,4'-diaminobenzophenone was subjected to structural analysis by measuring its ¹H-NMR and ²H-NMR spectra to show an average deuteration ratio of 77%.

### Example 13. Synthesis of deuterated polyamic acid compound

Deuterated 4,4'-diaminobenzophenone of 10 mmol obtained in Reference Example 7 and pyromellitic dianhydride of 10 mmol were added to N-methylpyrrolidone of 39 g and subjected to reaction at about 25°C for about 2 hours, followed by ordinary processing to obtain 4 g of a deuterated polyamic acid compound having the following repeating constitution (weight average molecular weight: 113,000) (yield: 93%).

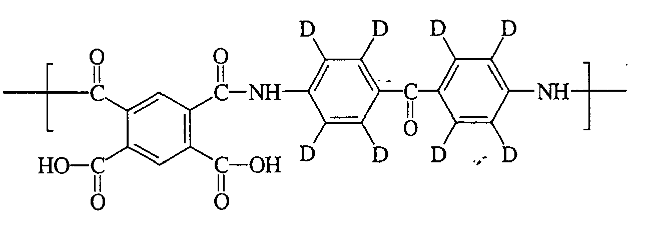

### Example 14. Synthesis of deuterated polyimide compound

1 g of 10% by weight N-methylpyrrolidone solution of the deuterated polyamic acid compound obtained in Example 13 was cast on a glass Petri dish, heated at about 200°C for about 1 hour and then subjected to reaction at about 300°C for about 1 hour to obtain 0.085 g of a deuterated polyimide compound having the following repeating constitution (yield: 85%).

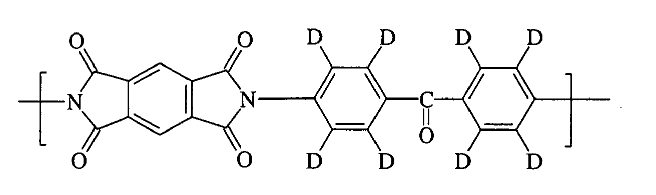

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Comparative Example 7.

A polyimide compound was obtained similarly as in Comparative Example 1 except for using 10 mmol of 4,4'-diaminobenzophenone not having a heavy hydrogen atom instead of 10 mmol of o-tolidine not having a heavy hydrogen atom (yield: 90%).

### Reference Example 8. Synthesis of deuterated 4,4'-diaminodiphenylsulfone

4,4'-diaminodiphenylsulfone of 10 g and a mixed catalyst of 3 g composed of 10% Pd/C of 1 g and 5% Pt/C of 2 g were added to deuterated water (D₂O) of 340 mL and subjected to reaction at about 180°C for 24 hours. After termination of the reaction, the reaction product was purified similarly as in Reference Example 1 to obtain deuterated 4,4'-diaminodiphenylsulfone of 9.6 g (yield: 96%). The obtained deuterated 4,4'-diaminodiphenylsulfone was subjected to structural analysis by measuring its ¹H-NMR and ²H-NMR spectra to show an average deuteration ratio of 47%.

### Example 15. Synthesis of deuterated polyamic acid compound

Deuterated 4,4'-diaminodiphenylsulfone of 10 mmol obtained in Reference Example 8 and pyromellitic dianhydride of 10 mmol were added to N-methylpyrrolidone of 42 g and subjected to reaction at about 25°C for about 2 hours, followed by ordinary processing to obtain 4 g of a deuterated polyamic acid compound having the following repeating constitution (weight average molecular weight: 105,000) (yield: 86%).

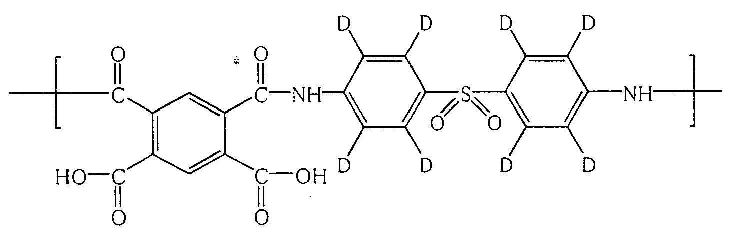

### Example 16. Synthesis of deuterated polyimide compound

1 g of 10% by weight N-methylpyrrolidone solution of the deuterated polyamic acid compound obtained in Example 15 was cast on a glass Petri dish, heated at about 200°C for about 1 hour and then subjected to reaction at about 300°C for about 1 hour to obtain 0.95 g of a deuterated polyimide compound having the following repeating constitution (yield: 95%).

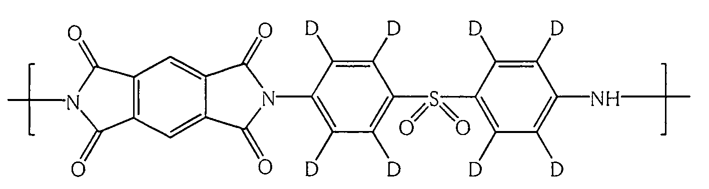

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Comparative Example 8.

A polyimide compound was obtained similarly as in Comparative Example 1 except for using 10 mmol of 4,4'-diaminodiphenylsulfone not having a heavy hydrogen atom instead of 10 mmol of o-tolidine not having a heavy hydrogen atom (yield: 90%).

### Reference Example 9. Synthesis of deuterated o-tolidine

o-Tolidine of 10 g and 5% Pt/C of 2 g were added to deuterated water (D₂O) of 340 mL and subjected to reaction at about 180°C for about 24 hours. After termination of the reaction, the reaction product was purified similarly as in Reference Example 1 to obtain deuterated o-tolidine of 6.5 g (yield: 65%). The obtained deuterated o-tolidine was subjected to structural analysis by measuring its ¹H-NMR and ²H-NMR spectra to show an average deuteration ratio of 30%.

### Example 17. Synthesis of deuterated polyamic acid compound

Deuterated o-tolidine of 10 mmol obtained in Reference Example 9 and pyromellitic dianhydride of 10 mmol were added to dimethylacetamide of 41 g and subjected to reaction at about 25°C for about 2 hours, followed by ordinary processing to obtain 4 g of a deuterated polyamic acid compound having the following repeating constitution (weight average molecular weight: 165,000) (yield: 88%).

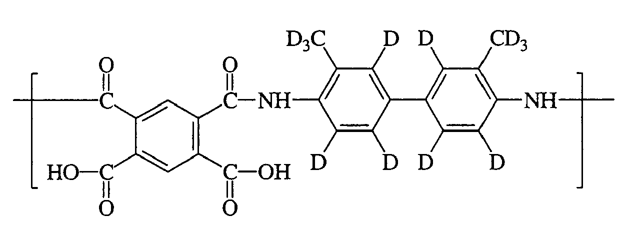

### Example 18. Synthesis of deuterated polyimide compound

1 g of 10% by weight dimethylacetamide solution of the deuterated polyamic acid compound obtained in Example 17 was cast on a glass Petri dish, heated at about 200°C for about 1 hour and then subjected to reaction at about 300°C for about 1 hour to obtain 0.095 g of a deuterated polyimide compound having the following repeating constitution (yield: 95%).

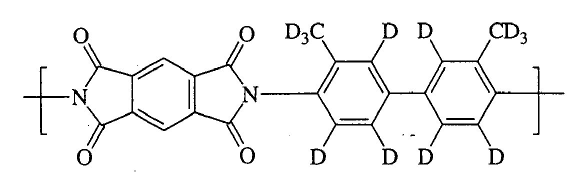

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 10. Synthesis of deuterated 4,4'-methylene di-o-toluidine

4,4'-methylene di-o-toluidine of 20 g (0.09 mol) and 5% Pt/C of 4 g were added to deuterated water (D₂O) of 680 mL and subjected to reaction at about 180°C for about 24 hours. After termination of the reaction, the reaction product was purified similarly as in Reference Example 1 to obtain deuterated 4,4'-methylene di-o-toluidine of 9 g (yield: 45%). The obtained deuterated 4,4'-methylene di-o-toluidine was subjected to structural analysis by measuring its ¹H-NMR and ²H-NMR spectra to show an average deuteration ratio of 81%.

### Example 19. Synthesis of deuterated polyamic acid compound

3.8 g of a deuterated polyamic acid compound (weight average molecular weight: 152,000) having the following repeating constitution was obtained similarly as in Example 3 except for using the deuterated 4,4'-methylene di-o-toluidine obtained in Reference Example 10 (yield: 95%).

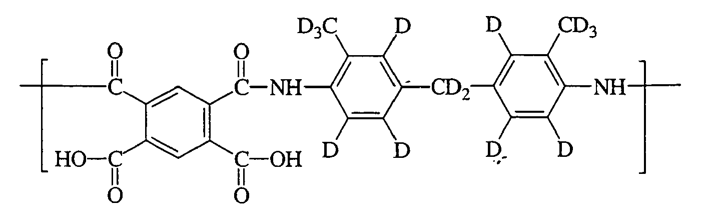

### Example 20. Synthesis of deuterated polyimide compound

1 g of 10% by weight N-methylpyrrolidone solution of the deuterated polyamic acid compound obtained in Example 19 was cast on a glass Petri dish, heated at about 200°C for about 1 hour and then subjected to reaction at about 300°C for about 1 hour to obtain 0.095 g of a deuterated polyimide compound having the following repeating constitution (yield: 95%).

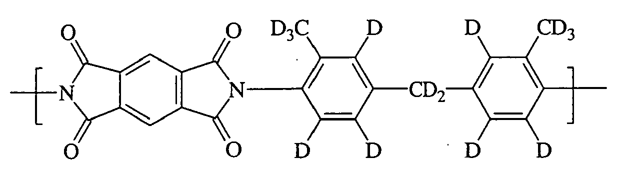

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 11. Synthesis of deuterated 3,3',5,5'-tetramethylbenzidine

3,3',5,5'-Tetramethylbenzidine of 10 g and 5% Pt/C of 2 g were added to deuterated water (D₂O) of 340 mL and subjected to reaction at 180°C for 24 hours. After termination of the reaction, the reaction product was purified similarly as in Reference Example 1 to obtain deuterated 3,3',5,5'-tetramethylbenzidine of 6.5 g (yield: 82%). The obtained deuterated 3,3',5,5'-tetramethylbenzidine was subjected to structural analysis by measuring its ¹H-NMR and ²H-NMR spectra to show an average deuteration ratio of 65%.

### Example 21. Synthesis of deuterated polyamic acid compound

3.6 g of a deuterated polyamic acid compound (weight average molecular weight: 118,000) having the following repeating constitution was obtained similarly as in Example 5 except for using the deuterated 3,3',5,5'-tetramethylbenzidine obtained in Reference Example 11 (yield: 90%).

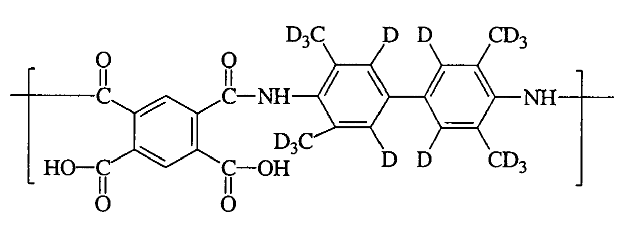

### Example 22. Synthesis of deuterated polyimide compound

1 g of 10% by weight N-methylpyrrolidone solution of the deuterated polyamic acid compound obtained in Example 21 was cast on a glass Petri dish; heated at about 200°C for about 1 hour and then subjected to reaction at about 300°C for about 1 hour to obtain 0.09 g of a deuterated polyimide compound having the following repeating constitution (yield: 90%).

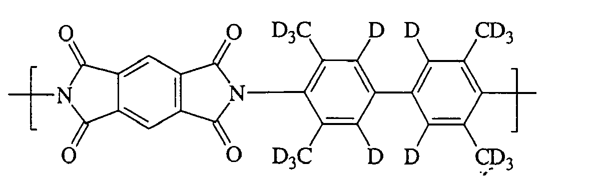

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 12. Synthesis of deuterated 4,4'-methylene di-2,6-xylidine

4,4'-Methylene di-2,6-xylidine of 10 g and 5% Pt/C of 2 g were added to deuterated water (D₂O) of 340 mL and subjected to reaction at about 180°C for about 24 hours. After termination of the reaction, the reaction product was purified similarly as in Reference Example 1 to obtain deuterated 4,4'-methylene di-2,6-xylidine of 8.2 g (yield: 82%). The obtained deuterated 4,4'-methylene di-2,6-xylidine was subjected to structural analysis by measuring its ¹H-NMR and ²H-NMR spectra to show an average deuteration ratio of 65%.

### Example 23. Synthesis of deuterated polyamic acid compound

3.6 g of a deuterated polyamic acid compound (weight average molecular weight: 132,000) having the following repeating constitution was obtained similarly as in Example 7 except for using the deuterated 4,4'-methylene di-2,6-xylidine obtained in Reference Example 12 (yield: 90%).

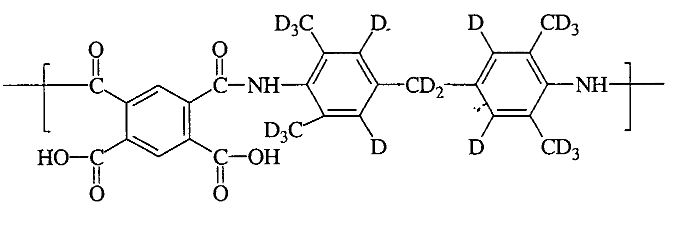

### Example 24. Synthesis of deuterated polyimide compound

1 g of 10% by weight N-methylpyrrolidone solution of the deuterated polyamic acid compound obtained in Example 23 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

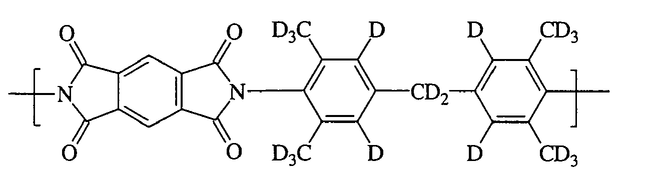

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 13. Synthesis of deuterated 4,4'-diaminodiphenylmethane

4,4'-Diaminodiphenylmethane of 10 g and 5% Pt/C of 2 g were added to deuterated water (D₂O) of 340 mL and subjected to reaction at about 180°C for about 24 hours. After termination of the reaction, the reaction product was purified similarly as in Reference Example 1 to obtain deuterated 4,4'-diaminodiphenylmethane of 8 g (yield: 80%). The obtained deuterated 4,4'-diaminodiphenylmethane was subjected to structural analysis by measuring its ¹H-NMR and ²H-NMR spectra to show an average deuteration ratio of 81%.

### Example 25. Synthesis of deuterated polyamic acid compound

Deuterated 4,4'-diaminodiphenylmethane of 10 mmol obtained in Reference Example 13 and pyromellitic dianhydride of 10 mmol were added to N-methylpyrrolidone of 36 g and subjected to reaction at about 25°C for about 2 hours, followed by ordinary processing to obtain 3.8 g of a deuterated polyamic acid compound having the following repeating constitution (weight average molecular weight: 139,000) (yield: 95%).

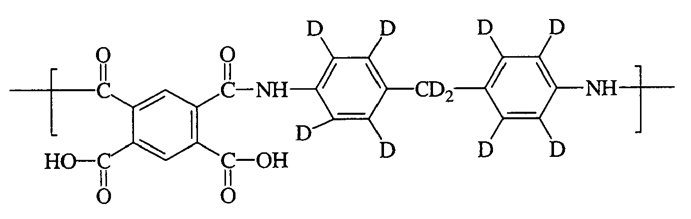

### Example 26. Synthesis of deuterated polyimide compound

1 g of 10% by weight dimethylacetamide solution of the deuterated polyamic acid compound obtained in Example 25 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

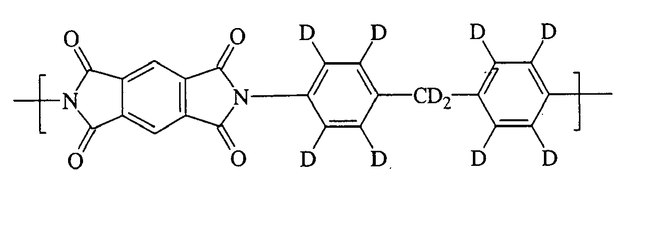

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 14. Synthesis of deuterated 4,4'-diaminodiphenyl ether

4,4'-Diaminodiphenyl ether of 10 g (0.09 mol) and 5% Pt/C of 2 g were added to deuterated water (D₂O) of 340 mL and subjected to reaction at about 180°C for about 24 hours. After termination of the reaction, the reaction product was purified similarly as in Reference Example 1 to obtain deuterated 4,4'-diaminodiphenyl ether of 9.5 g (yield: 95%). The obtained deuterated 4,4'-diaminodiphenyl ether was subjected to structural analysis by measuring its ¹H-NMR and ²H-NMR spectra to show an average deuteration ratio of 81%.

### Example 27. Synthesis of deuterated polyamic acid compound

3.6 g of a deuterated polyamic acid compound (weight average molecular weight: 179,000) having the following repeating constitution was obtained similarly as in Example 11 except for using the deuterated 4,4'-diaminodiphenyl ether obtained in Reference Example 14 (yield: 92%).

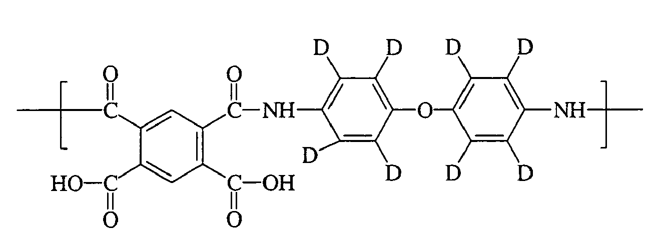

### Example 28. Synthesis of deuterated polyimide compound

1 g of 10% by weight N-methylpyrrolidone solution of the deuterated polyamic acid compound obtained in Example 27 was cast on a glass Petri dish, heated at about 200°C for about 1 hour and then subjected to reaction at about 300°C for about 1 hour to obtain 0.095 g of a deuterated polyimide compound having the following repeating constitution (yield: 95%).

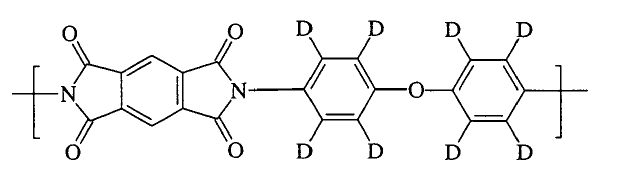

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 14. Synthesis of deuterated 4,4'-diaminobenzophenone

4,4'-Diaminobenzophenone of 10 g and 5% Pt/C of 2 g were added to deuterated water (D₂O) of 340 mL and subjected to reaction at 180°C for 24 hours. After termination of the reaction, the reaction product was purified similarly as in Reference Example 1 to obtain deuterated 4,4'-diaminobenzophenone of 6.5 g (yield: 82%). The obtained deuterated 4,4'-diaminobenzophenone was subjected to structural analysis by measuring its ¹H-NMR and ²H-NMR spectra to show an average deuteration ratio of 65%.

### Example 29. Synthesis of deuterated polyamic acid compound

4 g of a deuterated polyamic acid compound (weight average molecular weight: 113,000) having the following repeating constitution was obtained similarly as in Example 13 except for using the deuterated 4,4'-diaminobenzophenone obtained in Reference Example 14 (yield: 93%).

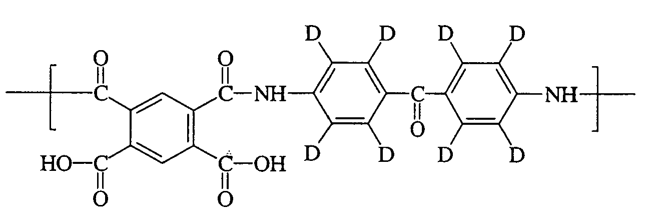

### Example 30. Synthesis of deuterated polyimide compound

1 g of 10% by weight N-methylpyrrolidone solution of the deuterated polyamic acid compound obtained in Example 29 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

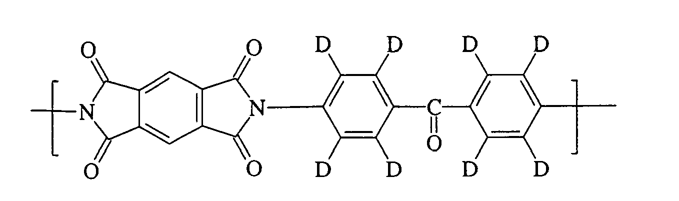

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 15. Synthesis of deuterated 4,4'-diaminodiphenylsulfone

4,4'-Diaminodiphenylsulfone of 10 g and 5% Pt/C of 2 g were added to deuterated water (D₂O) of 340 mL and subjected to reaction at about 180°C for about 24 hours. After termination of the reaction, the reaction product was purified similarly as in Reference Example 1 to obtain deuterated 4,4'-diaminodiphenylsulfone of 8.2 g (yield: 82%). The obtained deuterated 4,4'-diaminodiphenylsulfone was subjected to structural analysis by measuring its ¹H-NMR and ²H-NMR spectra to show an average deuteration ratio of 65%.

### Example 31. Synthesis of deuterated polyamic acid compound

3.8 g of a deuterated polyamic acid compound (weight average molecular weight: 108,000) having the following repeating constitution was obtained similarly as in Example 15 except for using the deuterated 4,4'-diaminodiphenylsulfone obtained in Reference Example 15 (yield: 82%).

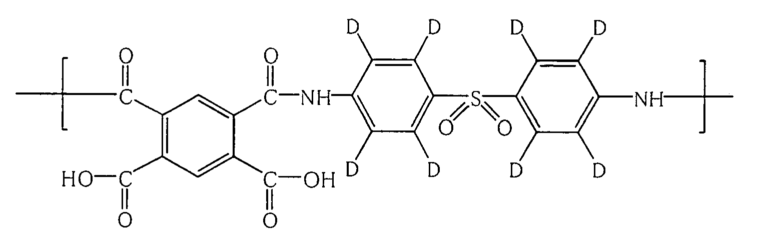

### Example 32. Synthesis of deuterated polyimide compound

1 g of 10% by weight N-methylpyrrolidone solution of the deuterated polyamic acid compound obtained in Example 31 was cast on a glass Petri dish, heated at about 200°C for about 1 hour and then subjected to reaction at about 300°C for about 1 hour to obtain 0.09 g of a deuterated polyimide compound having the following repeating constitution (yield: 90%).

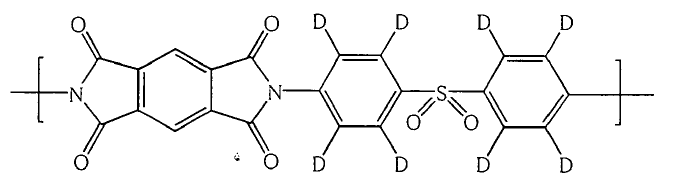

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained.

### Reference Example 16. Synthesis of deuterated 4,4'-methylene di-2,6-xylidine

4,4'-Methylene di-2,6-xylidine of 10 g and a mixed catalyst of 20 g composed of 1% Pd/C of 10 g and 1% Pt/C of 10 g were added to deuterated water (D₂O) of 340 mL and subjected to reaction at about 180°C for 24 hours. After termination of the reaction, the reaction solution was extracted with ethyl acetate, followed by filtering off the mixed catalyst. The obtained filtrate was dried using magnesium sulfate, concentrated under reduced pressure and then purified by column chromatography to obtain deuterated 4,4'-methylene di-2,6-xylidine of 9.3 g (yield: 93%). The obtained deuterated 4,4'-methylene di-2,6-xylidine was subjected to structural analysis by measuring its ¹H-NMR and ²H-NMR spectra to show an average deuteration ratio of 92%.

### Example 37. Synthesis of deuterated polyimide compound

Deuterated 4,4'-methylene di-2,6-xylidine of 5.7 g obtained in Reference Example 16 was dissolved in N-methylpyrrolidone of 80 ml under nitrogen stream and added with pyromellitic dianhydride of 5.5 g under cooling with ice, followed by stirring at room temperature for 2 hours. The reaction mixture was heated up to 180°C and stirred for 2 hours. After being left for cooling, the reaction mixture was added dropwise to methanol of 800 ml. The deposit separated by filtration was dried under reduced pressure to obtain 9.8 g of a deuterated polyimide compound (weight average molecular weight: 12,800) having the following repeating constitution as a light brown solid (yield: 94.1%).

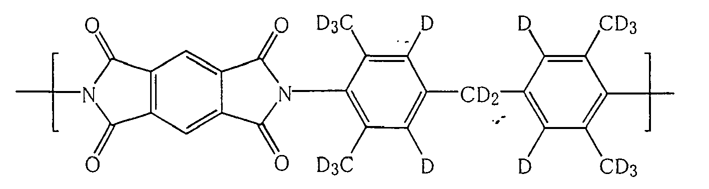

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 82%.

### Example 38. Synthesis of deuterated polyimide compound

12.6 g of a deuterated polyimide compound (weight average molecular weight: 16,000) having the following repeating constitution was obtained as a light brown solid in the similar operation as in Example 37 except for using 8.1 g of 3,3',4,4'-benzophenonetetracarboxylic dianhydride instead of pyromellitic dianhydride (yield: 97.1%).

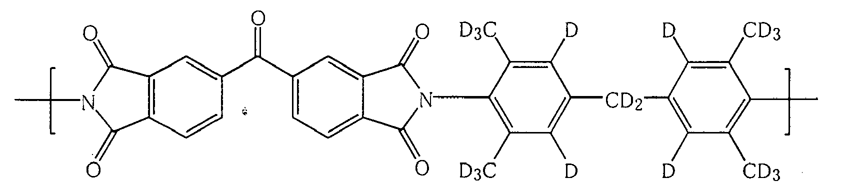

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 69%.

### Reference Example 17. Synthesis of deuterated 1,3-bis(3-aminophenoxy)benzene

1,3-bis(3-Aminophenoxy)benzene of 10 g and a mixed catalyst of 3 g composed of 10% Pd/C of 1 g and 5% Pt/C of 2 g were added to deuterated water (D₂O) of 340 mL and subjected to reaction at about 180°C for 24 hours. After termination of the reaction, the reaction solution was extracted with ethyl acetate, followed by filtering off the mixed catalyst. The obtained filtrate was dried using magnesium sulfate, concentrated under reduced pressure and then purified by column chromatography to obtain deuterated 1,3-bis(3-aminophenoxy)benzene of 9.8 g (yield: 98%). The obtained deuterated 1,3-bis(3-aminophenoxy)benzene was subjected to structural analysis by measuring its ¹H-NMR and ²H-NMR spectra to show an average deuteration ratio of 54%.

### Example 39. Synthesis of deuterated polyimide compound

10.8 g of a deuterated polyimide compound (weight average molecular weight: 13,800) having the following repeating constitution was obtained as a light brown solid in the similar operation as in Example 37 except for using 6.6 g of deuterated 1,3-bis(3-aminophenoxy)benzene obtained in Reference Example 17 (yield: 96.1%).

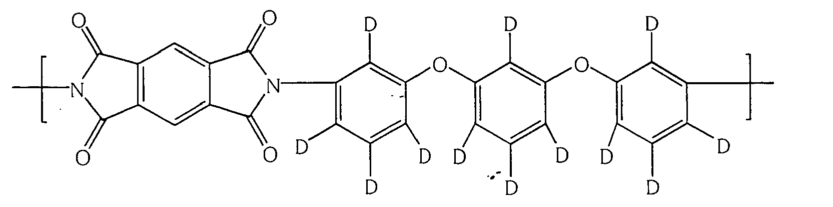

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 46%.

### Example 40. Synthesis of deuterated polyimide compound

9.4 g of a deuterated polyimide compound (weight average molecular weight: 12,000) having the following repeating constitution was obtained as a light brown solid in the similar operation as in Example 37 except that 5.1 g of deuterated o-tolidine obtained in Reference Example 1 was used (yield: 96.1%).

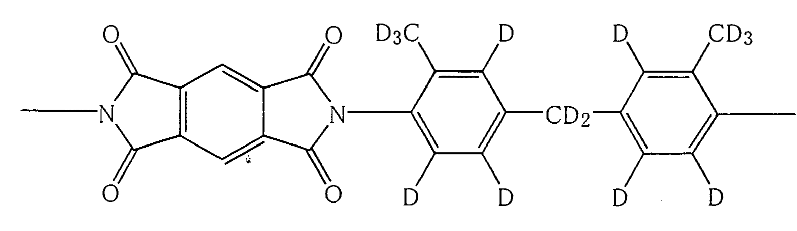

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 71%.

### Reference Example 18. Synthesis of deuterated 3,3'-diaminodiphenylsulfone

3,3'-Diaminodiphenylsulfone of 10 g and 5% Pt/C of 2 g were added to deuterated water (D₂O) of 340 mL and subjected to reaction at about 180°C for about 24 hours. After termination of the reaction, the reaction product was purified similarly as in Reference Example 1 to obtain deuterated 4,4'-diaminodiphenylsulfone of 9.2 g (yield: 92%). The obtained deuterated 4,4'-diaminodiphenylsulfone was subjected to structural analysis by measuring its ¹H-NMR and ²H-NMR spectra to show an average deuteration ratio of 48%.

### Example 41. Synthesis of deuterated polyimide compound

11.7 g of a deuterated polyimide compound (weight average molecular weight: 14,200) having the following repeating constitution was obtained as a light yellow solid in the similar operation as in Example 38 except for using the deuterated 2,2'-diaminodiphenylsulfone obtained in Reference Example 18 (yield: 91.3%).

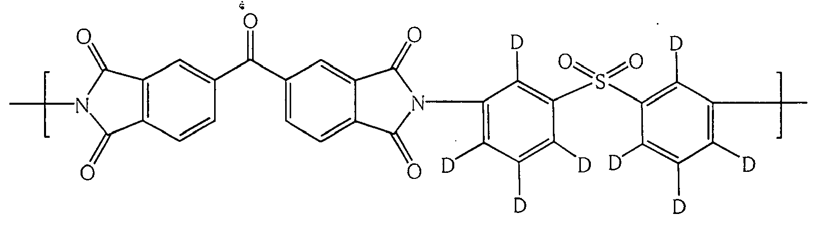

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 27%.

### Example 42. Synthesis of deuterated polyimide compound

12.5 g of a deuterated polyimide compound (weight average molecular weight: 15,800) having the following repeating constitution was obtained as a light yellow solid in the similar operation as in Example 41 except that the deuterated 4,4'-diaminodiphenylsulfone obtained in Reference Example 15 was used (yield: 97.7%).

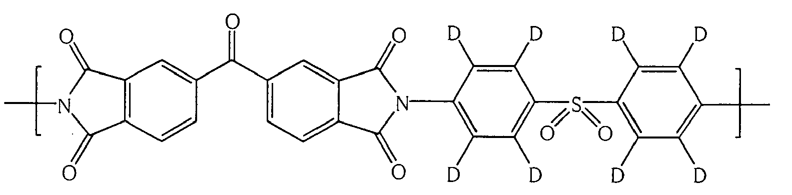

The obtained deuterated polyimide compound was subjected to structural analysis by measuring its IR spectrum, which showed peaks derived from C=O⁻ bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 37%.

### Example 43. Synthesis of deuterated polyimide compound

11.7 g of a deuterated polyimide compound (weight average molecular weight: 14,900) having the following repeating constitution was obtained as a light yellow solid in the similar operation as in Example 42 except for using 7.4 g of 3,4:3',4'-biphenyltracarboxylic dianhydride instead of pyromellitic dianhydride (yield: 96.1%).

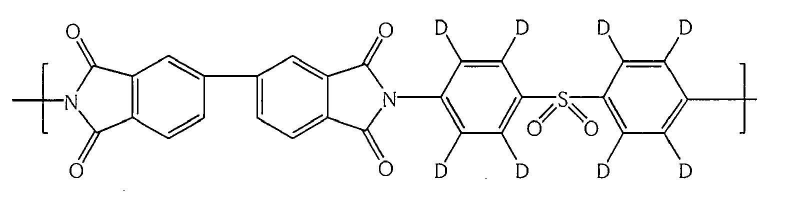

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 37%.

### Example 44. Synthesis of deuterated polyimide compound

12.3 g of a deuterated polyimide compound (weight average molecular weight: 16,200) having the following repeating constitution was obtained as a light yellow solid in the similar operation as in Example 43 except for using 6.6 g of the deuterated 1,3-bis(3-aminophenoxy)benzene obtained in Reference Example 17 (yield: 93.4%).

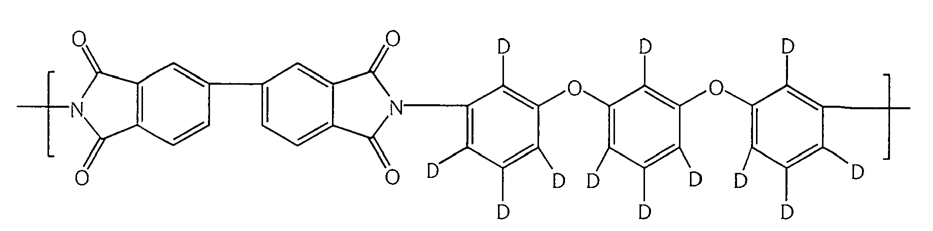

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 36%.

### Example 45. Synthesis of deuterated polyimide compound

12.0 g of a deuterated polyimide compound (weight average molecular weight: 15,400) having the following repeating constitution was obtained as a light yellow solid in the similar operation as in Example 43 except for using 7.8 g of the 3,3',4,4'-benzophenonetetracarboxylic dianhydride instead of 3,4:3',4'-biphenyltracarboxylic dianhydride (yield: 95.5%).

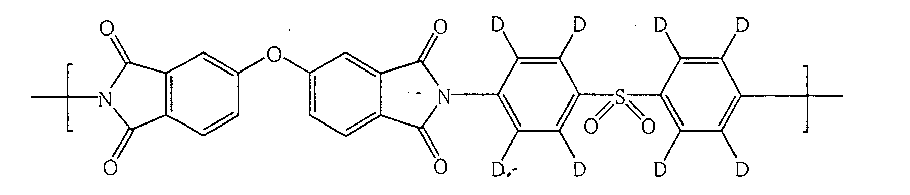

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 37%.

### Example 46. Synthesis of deuterated polyimide compound

10.5 g of a deuterated polyimide compound (weight average molecular weight: 14,100) having the following repeating constitution was obtained as a brown solid in the similar operation as in Example 45 except for using 4.5 g of the deuterated 4,4'-diaminodiphenyl ether obtained in Reference Example 6 (yield: 91.8%).

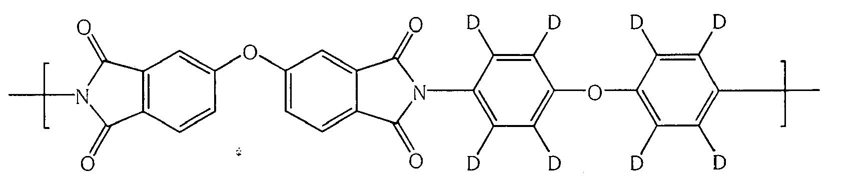

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its- ¹H-NMR and ²H-NMR spectra showed an average deuteration ratio of 56%.

### Example 47. Synthesis of deuterated polyimide compound

13.8 g of a deuterated polyimide compound (weight average molecular weight: 18,100) having the following repeating constitution was obtained as a light yellow solid in the similar operation as in Example 39 except for using 9.0 g of the 3,3',4,4'-diphenylsulfonetetracarboxylic dianhydride instead of pyromellitic dianhydride (yield: 93.8%).

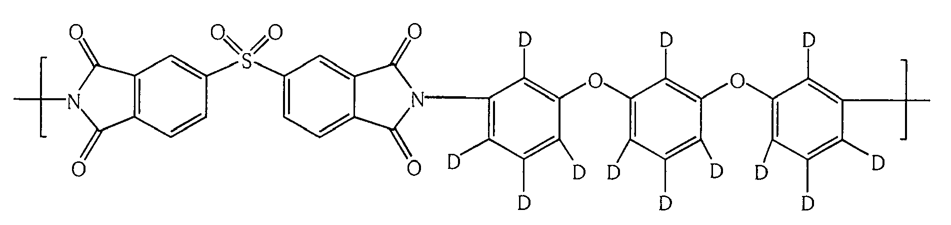

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 36%.

### Example 48. Synthesis of deuterated polyimide compound

7.2 g of a deuterated polyimide compound (weight average molecular weight: 11,900) having the following repeating constitution was obtained as a dark brown solid in the similar operation as in Example 42 except for using 4.9 g of the 1,2,3,4-butanetetracarboxylic dianhydride instead of 3,3',4,4'-benzophenonetetracarboxylic dianhydride (yield: 63.2%).

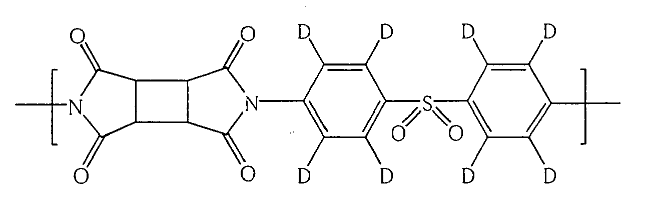

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 43%.

### Example 49. Synthesis of deuterated polyimide compound

9.8 g of a deuterated polyimide compound (weight average molecular weight: 12,500) having the following repeating constitution was obtained as a light brown solid in the similar operation as in Example 46 except for using 6.2 g of bicycle[2.2.2]oct-7-ene-2,3,5,6-tetracarboxylic dianhydride instead of 3,3',4,4'-benzophenonetetracarboxylic dianhydride (yield: 98.5%).

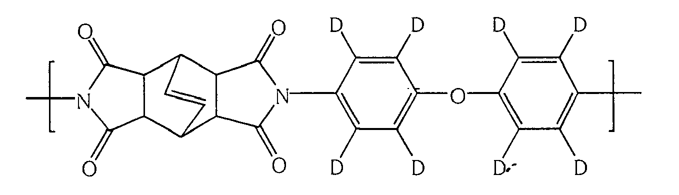

The obtained deuterated polyimide compound was subjected to structural analysis by measuring its IR spectrum, which showed peaks derived from C=O bonds and C-N bonds of the polyimide structure (around 1,730 cm⁻¹ and around 1,370 cm⁻¹ respectively). It was thus confirmed that the raw material deuterated polyamic acid compound was cyclized and the object deuterated polyimide compound was obtained. Structural analysis by measuring its ¹H-NMR and ²H-NMR spectra showed an average deuteration ratio of 49%.

Solutions prepared by dissolving the deuterated polyimide compounds obtained in the above Examples 37 to 49 in a solvent such as N-methyl-2-pyrrolidone can be applied according to an ordinary coating method to easily form a film of the above deuterated polyimide compounds.

The deuterated polyimide compounds obtained in the above Examples have extremely less C-H bonds compared with polyimide compounds that are not deuterated, and thus polymers derived from the above deuterated compounds have excellent transparency and a small optical transmission loss in a near-infrared region. When the deuterated polyimide compound relating to the present invention is used as a core material of an optical waveguide, it enables clad materials made of various raw materials to be used because of its high refractive index, without selecting a clad material having a refractive index matching the core material. Further, the deuterated polyimide compound relating to the present invention does not suffer reduction of surface tension because it does not have many fluorine atoms in the molecule, and thus has good adhesion to a base material or a substrate leading to good processability in coating and the like.

As mentioned above, the deuterated polyimide compound obtained by the method of the present invention is an extremely useful compound as a polymer material for an optical waveguide. The deuterated polyamic acid compound obtained by the method of the present invention can be said to be an important derivative or intermediate to produce the above deuterated polyimide compound.

### Industrial Applicability

The deuterated polyimide compound obtained by the method of the present invention has a very high deuteration ratio and thus can be used as a raw material of a polymer for an optical waveguide that has low moisture absorption, excellent heat resistance and transparency, a small optical transmission loss, a high refractive index and good adhesion to a base material or a substrate. An optical waveguide of high performance can be manufactured using the above deuterated polyimide compound.

## Claims

1. A method for producing a deuterated polyamic acid compound represented by the general formula [2]: 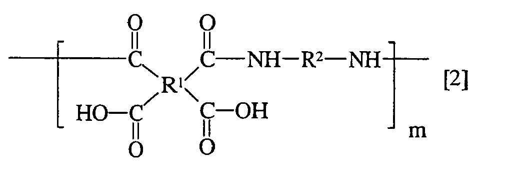 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and m indicates an integer not less than 1,
which comprises reacting an acid anhydride represented by the general formula [3]: 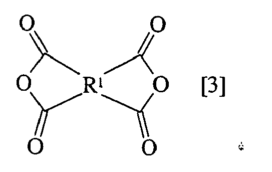 wherein R¹ has the same meaning as above,
with a deuterated diamine compound represented by the general formula [4]:
H₂N-R²-NH₂ [4]
wherein R² has the same meaning as above.

2. A method for producing a deuterated polyimide compound represented by the general formula [1]: 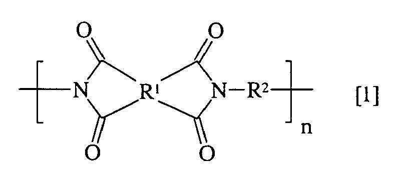 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
which comprises subjecting a deuterated polyamic acid compound represented by the general formula [2]: 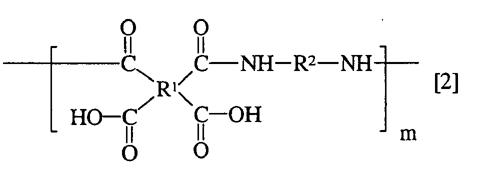 wherein R¹ and R² have the same meaning as above; and m indicates an integer not less than 1,
to a ring closure reaction.

3. The method according to claim 2, wherein the deuterated polyamic acid compound represented by the general formula [2] is obtained in the method according to claim 1.

4. A method for producing a deuterated polyimide compound represented by the general formula [1]: 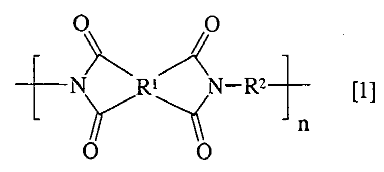 wherein, R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
which comprises reacting an acid anhydride represented by the general formula [3]: 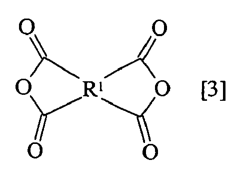 wherein R¹ has the same meaning as above;
and a deuterated diamine compound represented by the general formula [4]:
H₂N-R²-NH₂ [4]
wherein R² has the same meaning as above,
and then subjecting the reaction product to a ring closure reaction.

5. The method according to claim 1, wherein R¹ is a tetravalent aromatic hydrocarbon group which may have a heavy hydrogen atom.

6. The method according to claim 2, wherein R¹ is a tetravalent aromatic hydrocarbon group which may have a heavy hydrogen atom.

7. The method according to claim 4, wherein R¹ is a tetravalent aromatic hydrocarbon group which may have a heavy hydrogen atom.

8. The method according to claim 1, wherein the amount of heavy hydrogen atoms belonging to a divalent aromatic hydrocarbon group indicated by R² is not less than 20% of the amount of hydrogen atoms belonging to the divalent aromatic hydrocarbon.

9. The method according to claim 2, wherein the amount of heavy hydrogen atoms belonging to a divalent aromatic hydrocarbon group indicated by R² is not less than 20% of the amount of hydrogen atoms belonging to the divalent aromatic hydrocarbon.

10. The method according to claim 4, wherein the amount of heavy hydrogen atoms belonging toa divalent aromatic hydrocarbon group indicated by R² is not less than 20% of the amount of hydrogen atoms belonging to the divalent aromatic hydrocarbon.

11. The method according to claim 1, wherein a deuterated diamine compound represented by the general formula [4] is obtained by reacting the corresponding light hydrogen diamine compound with a heavy hydrogen source in the presence of a catalyst selected from an activated platinum catalyst, palladium catalyst, rhodium catalyst, ruthenium catalyst, nickel catalyst and cobalt catalyst.

12. The method according to claim 11, wherein the catalyst selected from an activated platinum catalyst, palladium catalyst, rhodium catalyst, ruthenium catalyst, nickel catalyst and cobalt catalyst is a platinum catalyst, a palladium catalyst or a mixed catalyst thereof.

13. The method according to claim 11, wherein the platinum catalyst is a platinum carbon and the palladium catalyst is a palladium carbon.

14. The method according to claim 11, wherein the heavy hydrogen source is deuterated water.

15. The method according to claim 4, wherein a deuterated diamine compound represented by the general formula [4] is obtained by reacting the corresponding light hydrogen diamine compound with a heavy hydrogen source in the presence of a catalyst selected from an activated platinum catalyst, palladium catalyst, rhodium catalyst, ruthenium catalyst, nickel catalyst and cobalt catalyst.

16. The method according to claim 15, wherein the catalyst selected from an activated platinum catalyst, palladium catalyst, rhodium catalyst, ruthenium catalyst, nickel catalyst and cobalt catalyst is a platinum catalyst, a palladium catalyst or a mixed catalyst thereof.

17. The method according to claim 15, wherein the platinum catalyst is a platinum carbon and the palladium catalyst is a palladium carbon.

18. The method according to claim 15, wherein the heavy hydrogen source is deuterated water.

19. Use of a deuterated polyimide compound represented by the general formula [1]: 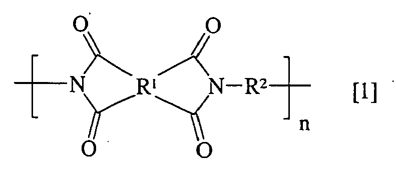 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
which is obtained in the method according to claim 2, as a raw material of a polymer for an optical waveguide.

20. Use of a deuterated polyimide compound represented by the general formula [1]: 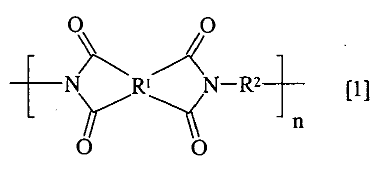 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
which is obtained in the method according to claim 4, as a raw material of a polymer for an optical waveguide.

21. A film for an optical waveguide containing a deuterated polyimide compound represented by the general formula [1]: 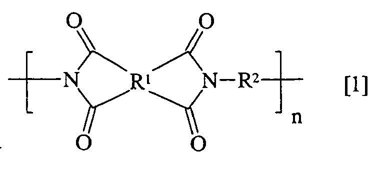 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
which is obtained in the method according to claim 2.

22. A film for an optical waveguide containing a deuterated polyimide compound represented by the general formula [1]: 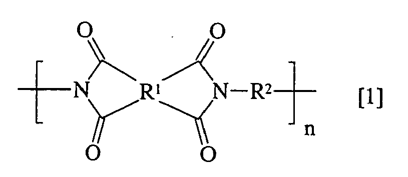 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1,
which is obtained in the method according to claim 4.

23. A deuterated polyimide compound represented by the general formula [1]: 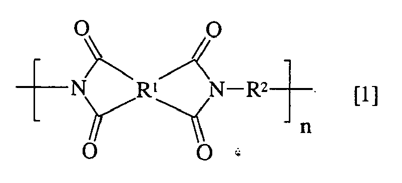 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and n indicates an integer not less than 1.

24. A deuterated polyamic acid compound represented by the general formula [2]: 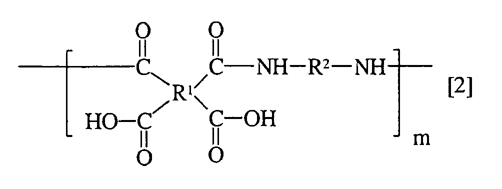 wherein R¹ indicates a tetravalent alicyclic hydrocarbon group or a tetravalent aromatic hydrocarbon group, which may have a heavy hydrogen atom; R² indicates a divalent aromatic hydrocarbon group having a heavy hydrogen atom; and m indicates an integer not less than 1.

25. The deuterated polyimide compound according to claim 23, wherein R¹ indicates a tetravalent aromatic hydrocarbon group which may have a heavy hydrogen atom.

26. The deuterated polyimide compound according to claim 23, wherein the deuterated polyimide compound represented by the general formula [1] is a compound represented by the following general formula [1'^{'}]: 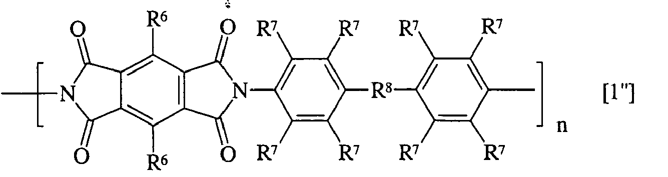 wherein R⁶ s indicate each independently a heavy hydrogen atom or a light hydrogen atom; R⁷ s indicate each independently a light hydrogen atom, a heavy hydrogen atom or a methyl group which may be deuterated; R⁸ indicates a direct-linkage or a methylene group which may be deuterated; and n indicates an integer not less than 1; and provided that the following Chemical Formula of the partial structure is deuterated:

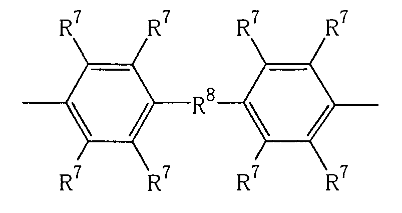

27. The deuterated polyamic acid compound according to claim 24, wherein the deuterated polyamic acid compound represented by the general formula [2] is a compound represented by the following general formula [2'^{'}] : 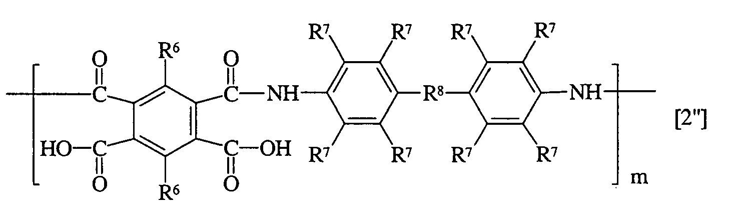 wherein R⁶ s indicate each independently a heavy hydrogen atom or a light hydrogen atom; R⁷ s indicate each independently a heavy hydrogen atom or a methyl group which is deuterated; R⁸ indicates a direct-linkage or a methylene group which is deuterated; and m indicates an integer not less than 1, and provided that the following Chemical Formula of the partial structure is deuterated).
